# EUROPÄISCHE PATENTSCHRIFT

(11) **EP 3 548 473 B1**
(45) Veröffentlichungstag und Bekanntmachung des Hinweises auf die Patenterteilung: **18.11.2020**
(21) Anmeldenummer: 17808461.2
(22) Anmeldetag: 01.12.2017
(51) Int. Cl.: C07D 307/46

(54) **HMF-HERSTELLUNGSVERFAHREN**
PROCESS FOR PREPARING HMF
PROCEDE DE PREPARATION DU HMF

(30) Priorität: 02.12.2016 DE 102016224073
(43) Veröffentlichungstag der Anmeldung: 09.10.2019
(73) Patentinhaber: Südzucker AG, 68165 Mannheim (DE)
(72) Erfinder: KUNZ, Markwart, 38106 Braunschweig (DE); HAJI BEGLI, Alireza, 67305 Ramsen (DE); KRÖNER, Christine, 67283 Obrigheim (DE); WACH, Wolfgang, 67549 Worms (DE); GRAF, Alain-Michel, 67549 Worms (DE); KRAUS, Wolfgang, 69226 Nußloch (DE)
(74) Vertreter: Schrell, Andreas
(86) Internationale Anmeldenummer: PCT/EP2017/081236
(87) Internationale Veröffentlichungsnummer: WO 2018/100184

(56) Entgegenhaltungen:
- EP-A2- 0 230 250
- WO-A2-2015/113060

## Beschreibung

Die vorliegende Erfindung betrifft ein Verfahren zur Herstellung von 5-Hydroxymethylfurfural (HMF) in einem kontinuierlichen Prozess, der zum Erhalt einer HMF-Fraktion, einer Kohlenhydrat/Säure-Fraktion, einer Fructose-Fraktion und einer Lävulin- und Ameisensäure-Fraktion führt und es aufgrund der Reinheit der erhaltenen Fraktionen vorteilhafterweise ermöglicht, die mit dem Verfahren erhaltene Fructose-Fraktion direkt in den Herstellungsprozess zurückzuführen und die weiteren Fraktionen ohne die Notwendigkeit aufwendiger, zusätzlicher Aufreinigungsschritte in weiterverarbeitenden Prozessen zu verwenden.

5-Hydroxymethylfurfural (HMF) ist ein multifunktionales Molekül mit einem aromatischen 5-Ring-System, einer Aldehyd- und einer Alkoholgruppe. Die vielen Funktionalitäten machen das Molekül zu einer vielfältig einsetzbaren Plattformchemikalie, die als Basis für eine große Vielzahl weiterer Verbindungen dienen kann. Zu den Verbindungen, die auf Basis von HMF hergestellt werden können, gehören zum einen Chemikalien, die heute bereits im Bulkmaßstab auf petrochemischem Wege hergestellt werden, beispielsweise Caprolactam oder Adipinsäure, aber auch Verbindungen mit einem großen Anwendungspotential, für die bislang noch kein technischer Herstellprozess zur Verfügung steht, wie 2,5-Furandicarbonsäure (FDCA).

Trotz des großen Potentials von HMF und FDCA fehlt es bislang an wirtschaftlichen, technisch etablierten Herstellungsverfahren für diese Verbindungen. Die Multifunktionalität von HMF als einer der größten Vorteile des Moleküls hat sich in Bezug auf die daraus resultierende mögliche Folgechemie gleichfalls als Hauptnachteil bei der Synthese erwiesen. HMF ist vor allem in wässrigen Systemen unter den für die Synthese notwendigen Reaktionsbedingungen (saurer pH-Wert, erhöhte Temperatur) nicht stabil und reagiert zum einen unter Polymerisation mit sich selbst und/oder den Edukten und Zwischenprodukten zu sogenannten Huminen, die je nach Kettenlänge löslich oder unlöslich sind und zu einer Braun- bis Schwarzfärbung der Reaktionslösung führen. Eine weitere unerwünschte Folgereaktion ist die saure Hydrolyse von HMF zu Lävulin- und Ameisensäure, wobei insbesondere Lävulinsäure mit HMF zu weiteren unerwünschten Nebenprodukten reagieren kann. Für eine möglichst wirtschaftliche Herstellung von HMF ist es daher unbedingt notwendig, das Auftreten dieser Nebenreaktion sowie die Folgereaktion von HMF und Lävulinsäure soweit wie möglich zu vermeiden.

Grundsätzlich kann bei den vielzähligen unterschiedlichen Synthesewegen, die im Stand der Technik zur Herstellung von HMF beschrieben wurden, zwischen einphasigen und zweiphasigen Reaktionssystemen unterschieden werden. Bei beiden Ansätzen können sowohl homogene als auch heterogene Katalysatoren zum Einsatz kommen. Bei den einphasigen Systemen kann die HMF-Synthese neben rein wässrigen Systemen auch in organischen Lösungsmitteln, wie beispielsweise DMSO, DMF und Sulfolan, oder in ionischen Flüssigkeiten durchgeführt werden. Die Vermeidung von wässrigen Systemen führt zwar rein auf die chemische Reaktion bezogen zu besseren Selektivitäten für HMF, jedoch sind für die Entfernung der Lösungsmittel oftmals hohe Temperaturen notwendig, unter denen es zur thermischen Zersetzung von HMF kommen kann, wodurch wiederum die Reinheit und Ausbeute des HMF deutlich verschlechtert wird. Zudem spielen bei der Verwendung von wasserfreien Systemen die Kosten für die Lösungsmittel sowie Sicherheits- und Umweltaspekte eine große Rolle. Auch erweist sich als nachteilig, dass die für die HMF-Synthese eingesetzten Hexosen, insbesondere Fructose und/oder Glukose, in vielen gängigen organischen Lösungsmitteln eine schlechte Löslichkeit aufweisen.

Bei den zweiphasigen Reaktionssystemen wird die Reaktion der Hexose zu HMF in wässriger Phase durchgeführt und das entstandene HMF kontinuierlich mithilfe eines organischen Lösungsmittels extrahiert. Dabei darf das Lösungsmittel nicht mit Wasser vermischbar sein und muss einen ausreichend hohen Verteilungskoeffizienten für HMF zwischen wässriger und organischer Phase aufweisen, um eine effiziente Extraktion des HMF zu gewährleisten. Da insbesondere die Verteilungskoeffizienten für die meisten Lösungsmittel nicht sehr hoch sind, müssen in solchen Systemen oftmals sehr große Mengen an Lösungsmittel eingesetzt werden. Das in zweiphasigen Reaktionssystemen am häufigsten verwendete organische Lösungsmittel ist dabei Methylisobutylketon (MIBK), welches gegebenenfalls in Kombination mit Phasenmodifizierern wie 2-Butanol eingesetzt wird. Wie bereits für die einphasigen wasserfreien Reaktionssysteme dargestellt, erweist sich jedoch auch in diesem Fall die abschließende Entfernung der oder des verwendeten Lösungsmittel(s) aufgrund der hohen Siedepunkte geeigneter Lösungsmittel als problematisch.

EP 0 230 250 B1 offenbart Verfahren zur Herstellung von 5-Hydroxymethylfurfural einschließlich eines kristallinen Produktes unter alleiniger Verwendung von Wasser als Lösungsmittel. In dem beschriebenen Batch-Verfahren werden Saccharide in wässriger Lösung bei einer Temperatur von über 100 °C mit einem sauren Katalysator zu einem Gemisch von Hexosen und HMF zersetzt und anschließend das gebildete HMF über Ionenaustauschersäulen bei einer Temperatur von 35 bis 85 °C von Nebenprodukten so getrennt, dass neben einer HMF-Fraktion eine Saccharid-Fraktion gewonnen werden kann, die für eine erneute HMF-Synthese gemäß dem beschriebenen Verfahren zur Verfügung steht. Die in diesem Dokument offenbarte batchweise Umsetzung geht mit einem hohen Fructoseumsatz und einer daraus unmittelbar folgenden hohen HMF-Konzentration in der Reaktionslösung einher, die unter den vorherrschenden Bedingungen zu einer vermehrten Bildung von Neben- und Abbauprodukten führt, wodurch es in Bezug auf die umgesetzte Fructosemenge zu einer verringerten HMF-Ausbeute kommt.

WO 2013/106136 A1 betrifft ein Verfahren zur Herstellung von HMF und HMF-Derivaten aus Zucker, umfassend die Rückgewinnung von nicht umgesetzten Zuckern, die zur direkten Verwendung in der Ethanol-Fermentation geeignet sind. Dabei werden Hexose-haltige Lösungen in wässriger Phase durch säurekatalysierte Dehydratisierungsreaktion zu HMF umgesetzt, anschließend werden die in der Produktmischung enthaltenen nicht umgesetzten Zucker durch Adsorption und/oder Lösungsmittelextraktion aus dem Produktmischung abgetrennt und diese schließlich in aeroben oder anaeroben Fermentationsverfahren zur Ethanolgewinnung eingesetzt. Es wird gelehrt, die säurekatalysierte Dehydratisierungsreaktion bei einer Temperatur von 175 bis 205 °C durchzuführen.

WO 2015/113060 A2 offenbart die Umwandlung von Fructose-haltigen Ausgangsmaterialien zu HMF-haltigen Produkten. Mittels des beschriebenen Verfahrens werden Fructose, Wasser, ein Säurekatalysator und mindestens ein weiteres Lösungsmittel in einer Reaktionszone vermischt und durch Wahl geeigneter Reaktionsparameter für eine Dauer von ca. 1 bis 60 Minuten zur Reaktion gebracht, sodass eine HMF-Ausbeute von 80 % nicht überschritten wird. Beim Erreichen des festgelegten Umsatzes werden die Reaktionskomponenten umgehend abgekühlt, um die Bildung von unerwünschten Nebenprodukten zu minimieren.

Zur Sicherstellung eines kostengünstigen und effektiven Herstellungsverfahrens für HMF ist entscheidend, dass während der Umsetzung einer Fructose-haltigen Ausgangslösung zu HMF die Bildung von unerwünschten Nebenprodukten und die Zersetzung des in der säurekatalysierten Dehydratisierungsreaktion gebildeten HMF durch die Wahl geeigneter Reaktionsbedingungen und Verfahrensschritte soweit wie möglich vermieden wird und nicht umgesetzte Fructose abgetrennt von den während der Dehydratisierungsreaktion gebildeten störenden Nebenprodukten und somit in möglichst reiner Form für die Rückführung in den kontinuierlichen Herstellungsprozess bereitgestellt wird.

Ein entsprechendes Verfahren zur kostengünstigen und effektiven Herstellung von HMF in einem kontinuierlichen Prozess ist bislang aus dem Stand der Technik nicht bekannt.

Es ist daher die Aufgabe der vorliegenden Erfindung, die genannten Nachteile und Einschränkungen der aus dem Stand der Technik bekannten Verfahren zu überwinden, insbesondere ein Verfahren bereitzustellen, Fructose unter Säurekatalyse hochselektiv, insbesondere unter maximaler Vermeidung von Nebenproduktbildung und auf kostengünstige und effektive Weise, zu HMF umzusetzen.

Die Aufgabe der vorliegenden Erfindung wird insbesondere durch die technische Lehre der unabhängigen Ansprüche gelöst.

Die vorliegende Erfindung betrifft insbesondere ein Verfahren zur Herstellung von 5-Hydroxymethylfurfural (HMF) in einem kontinuierlichen Prozess, umfassend die Schritte:
a) Bereitstellen einer wässrigen Fructose-haltigen Ausgangslösung und mindestens eines homogenen sauren Katalysators,
b) Vermischen der wässrigen Fructose-haltigen Ausgangslösung und des mindestens einen homogenen sauren Katalysators zum Erhalt einer Reaktionslösung mit einem Kohlenhydratgehalt von 5 Gew.-% bis 50 Gew.-% (Trockensubstanz Kohlenhydrat in Bezug auf Gesamtgewicht Reaktionslösung) und einem Fructosegehalt von 40 Gew.-% bis 100 Gew.-% (Trockensubstanz Fructose in Bezug auf Trockensubstanz der Kohlenhydrate),
c) Einspeisen der in Schritt b) erhaltenen Reaktionslösung in ein kontinuierliches Reaktorsystem und Umsetzen der in der Reaktionslösung vorhandenen Fructose zu HMF bei einer Temperatur von 80 °C bis 165 °C zum Erhalt einer HMF-haltigen Produktmischung unter Einstellung eines Fructose-Umsatzes von 1 mol-% bis 40 mol-%,
d) Einstellen der Produktmischung auf eine Temperatur von 20 °C bis 80 °C, und
e) Aufreinigen der in Schritt d) erhaltenen Produktmischung unter Verwendung einer Chromatographie zur Auftrennung von mindestens vier Fraktionen, umfassend eine HMF-Fraktion, eine Kohlcnhydrat/Säurc-Fraktion, eine Fructosc-Fraktion und eine Lävulin- und Ameisensäure-Fraktion.

In einer bevorzugten Ausführungsform der vorliegenden Erfindung umfasst das vorstehende Verfahren zur Herstellung von 5-Hydroxymethylfurfural (HMF) in einem weiteren Schritt f) das Erhalten mindestens einer HMF-Fraktion, einer Kohlenhydrat/Säure-Fraktion, einer Fructose-Fraktion und einer Lävulin- und Ameisensäure-Fraktion.
Die vorliegende Erfindung betrifft insbesondere ein Verfahren zur Herstellung von 5-Hydroxymethylfurfural (HMF) in einem kontinuierlichen Prozess, umfassend die Schritte:
a) Bereitstellen einer wässrigen Fructose-haltigen Ausgangslösung und mindestens eines homogenen sauren Katalysators,
b) Vermischen der wässrigen Fructose-haltigen Ausgangslösung und des mindestens einen homogenen sauren Katalysators zum Erhalt einer Reaktionslösung mit einem Kohlenhydratgehalt von 5 Gew.-% bis 50 Gew.-% (Trockensubstanz Kohlenhydrat in Bezug auf Gesamtgewicht Reaktionslösung) und einem Fructosegehalt von 40 Gew.-% bis 100 Gew.-% (Trockensubstanz Fructose in Bezug auf Trockensubstanz der Kohlenhydrate),
c) Einspeisen der in Schritt b) erhaltenen Reaktionslösung in ein kontinuierliches Reaktorsystem und Umsetzen der in der Reaktionslösung vorhandenen Fructose zu HMF bei einer Temperatur von 80 °C bis 165 °C zum Erhalt einer HMF-haltigen Produktmischung unter Einstellung eines Fructose-Umsatzes von 1 mol-% bis 40 mol-%,
d) Einstellen der Produktmischung auf eine Temperatur von 20 °C bis 80 °C,
e) Aufreinigen der in Schritt d) erhaltenen Produktmischung unter Verwendung einer Chromatographie zur Auftrennung von mindestens vier Fraktionen, umfassend eine HMF-Fraktion, eine Kohlenhydrat/Säure-Fraktion, eine Fructose-Fraktion und eine Lävulin- und Ameisensäure-Fraktion und
f) Erhalten mindestens einer HMF-Fraktion, einer Kohlenhydrat/Säure-Fraktion, einer Fructose-Fraktion und einer Lävulin- und Ameisensäure-Fraktion.

In einer bevorzugten Ausführungsform betrifft die vorliegende Erfindung ein Verfahren zur Herstellung von 5-Hydroxymethylfurfural (HMF) in einem kontinuierlichen Prozess, umfassend die Schritte:
a) Bereitstellen einer wässrigen Fructose-haltigen Ausgangslösung, einer wässrigen rückgeführten Fructose-haltigen Fraktion und mindestens eines homogenen sauren Katalysators,
b) Vermischen der wässrigen Fructose-haltigen Ausgangslösung, der wässrigen rückgeführten Fructose-haltigen Fraktion und des mindestens einen homogenen sauren Katalysators zum Erhalt einer Reaktionslösung mit einem Kohlenhydratgehalt von 5 Gew.-% bis 50 Gew.-% (Trockensubstanz Kohlenhydrat in Bezug auf Gesamtgewicht Reaktionslösung) und einem Fructosegehalt von 40 Gew.-% bis 100 Gew.-% (Trockensubstanz Fructose in Bezug auf Trockensubstanz der Kohlenhydrate),
c) Einspeisen der in Schritt b) erhaltenen Reaktionslösung in ein kontinuierliches Reaktorsystem und Umsetzen der in der Reaktionslösung vorhandenen Fructose zu HMF bei einer Temperatur von 80 °C bis 165 °C zum Erhalt einer HMF-haltigen Produktmischung unter Einstellung eines Fructose-Umsatzes von 1 mol-% bis 40 mol-%,
d) Einstellen der Produktmischung auf eine Temperatur von 20 °C bis 80 °C,
e) Aufreinigen der in Schritt d) erhaltenen Produktmischung unter Verwendung einer Chromatographie zur Auftrennung von mindestens vier Fraktionen, umfassend eine HMF-Fraktion, eine Kohlenhydrat/Säure-Fraktion, eine Fructose-Fraktion und eine Lävulin- und Ameisensäure-Fraktion, wobei die erhaltene Fructose-Fraktion kontinuierlich zumindest teilweise in den Schritt a) rückgeführt wird.

In einer bevorzugten Ausführungsform der vorliegenden Erfindung umfasst das vorstehende Verfahren zur Herstellung von 5-Hydroxymethylfurfural (HMF) in einem weiteren Schritt f) das Erhalten mindestens einer HMF-Fraktion, einer Kohlenhydrat/Säure-Fraktion, einer Fructose-Fraktion und einer Lävulin- und Ameisensäure-Fraktion, wobei die erhaltene Fructose-Fraktion kontinuierlich zumindest teilweise in den Schritt a) rückgcführt wird.

Erfindungsgemäß wird demnach ein Verfahren bereitgestellt, welches 5-Hydroxymethylfurfural (HMF) in einem kontinuierlichen Prozess durch selektive, bevorzugt hochselektive, säurekatalysierte Umsetzung der Fructose einer wässrigen Fructose-haltigen Ausgangslösung und in bevorzugter Ausführungsform einer aus dem Verfahren gewonnenen rückgeführten Fructose-haltigen Fraktion herstellt. Das erfindungsgemäße Verfahren zur Herstellung von HMF wird erfindungsgemäß so geführt, dass es in dem in Schritt c) eingesetzten kontinuierlichen Reaktorsystem durch Einstellung der Temperatur, und bevorzugt auch der Reaktionszeit, zielgerichtet zu einer limitierten Umsetzung der Fructose von 1 mol-% bis 40 mol-% kommt, wodurch eine überraschend hohe HMF-Selektivität erreicht werden kann. Nach Erreichen des limitierten Fructose-Umsatzes von maximal 40 mol-% wird die erhaltene Produktmischung in Schritt d) auf eine Temperatur von 20 °C bis 80 °C eingestellt, so dass die Bildung unerwünschter Nebenprodukte sowie die Zersetzung des gebildeten HMF weitestgehend verhindert wird. In einem folgenden Schritt e) wird das in der Produktmischung enthaltene HMF unter Verwendung eines chromatographischen Verfahrens, insbesondere mittels Chromatographie an Ionenaustauscherharzen, insbesondere Kationenaustauscherharzen, insbesondere mittels ein- oder mehrstufiger Chromatographie an Ionenaustauscherharzen, insbesondere Kationenaustauscherharzen, von den weiteren Komponenten der Produktmischung abgetrennt. Bevorzugt wird in einem anschließend durchgeführten Schritt f), also nach Durchführung des chromatographischen Verfahrens, neben der HMF-Fraktion eine Kohlenhydrat/Säure-Fraktion, eine Fructose-Fraktion und eine Lävulin- und Ameisensäure-Fraktion erhalten, insbesondere isoliert. Vorteilhafterweise weisen die über das verwendete chromatographische Verfahren erhaltenen einzelnen Fraktionen derart hohe Reinheiten auf, dass sie direkt, gegebenenfalls nach Aufkonzentrierung, das heißt ohne weitere Aufreinigung, in unterschiedlichen Folgeprozessen eingesetzt werden können.

Erfindungsgemäß bevorzugt ist die erhaltene Fructose-Fraktion weitgehend frei, insbesondere vollständig frei, von gebildeter Lävulinsäure. Erfindungsgemäß bevorzugt ist die erhaltene Fructose-Fraktion weitgehend frei, insbesondere vollständig frei von gebildeter Lävulin- und Ameisensäure.

Lävulinsäure begünstigt nachteiligerweise die Huminstoffbildung während der HMF-Synthese. So würde ein über die gemäß einer bevorzugten Ausführungsform rückgeführte wässrige Fructose-Fraktion bewirkter erhöhter Gehalt an Lävulinsäure in der Reaktionslösung zu einer vermehrten Bildung von Huminstoffen aus HMF und Kohlenhydraten führen und damit die Wirtschaftlichkeit des Prozesses deutlich herabsetzen. Die in dem erfindungsgemäßen Verfahren in Schritt e) aufgetrennte, bevorzugt in Schritt f) erhaltene, Fructose-Fraktion weist jedoch vorteilhafterweise eine derart hohe Reinheit auf, ist insbesondere frei von gebildeter Lävulinsäure, besonders bevorzugt frei von Lävulin- und Ameisensäure, dass sie in einer bevorzugten Ausführungsform direkt, gegebenenfalls nach Aufkonzentrierung, insbesondere ohne Reinigungsschritte, zur weiteren Umsetzung in das Verfahren, insbesondere Schritt a), zurückgeführt werden kann. Insbesondere kommt es durch die nach dem erfindungsgemäßen Verfahren vorgesehene limitierte Umsetzung von Fructose und damit verbunden der verringerten Bildung von Neben- und Abbauprodukten, insbesondere Lävulin- und Ameisensäure und Huminstoffen, sowie in bevorzugter Ausführungsform durch die Rückführung einer vom Produktgemisch abgetrennten Fraktion nicht umgesetzter Fructose zur einer hohen HMF-Selektivität und einer hohen HMF-Ausbeute.

Erfindungsgemäß umfasst das vorliegende Verfahren die Schritte a) bis e), bevorzugt a) bis f). In bevorzugter Ausführungsform kann das Verfahren daher neben den Schritten a) bis e), besonders bevorzugt a) bis f), auch weitere Verfahrensschritte umfassen, beispielsweise mindestens einen Filtrationsschritt, mindestens einen Entfärbungs- und/oder Reinigungsschritt, zum Beispiel mittels Aktivkohle, und/oder mindestens einen Aufkonzentrierungsschritt. In besonders bevorzugter Ausführungsform besteht das vorliegende Verfahren aus den Verfahrensschritten a) bis e), bevorzugt a) bis f). In bevorzugter Ausführungsform wird das Verfahren in der Reihenfolge der Verfahrensschritte a), b), c), d) und e), bevorzugt a), b), c), d), e) und f) durchgeführt.

Erfindungsgemäß erfolgt im Verfahren zur Herstellung von 5-Hydroxymethylfurfural gemäß der Schritte a) bis e), bevorzugt a) bis f), die Umsetzung der in der Reaktionsmischung vorhandenen Fructose zu HMF in einem kontinuierlichen Reaktorsystem und die anschließende Aufreinigung der erhaltenen Produktmischung unter Verwendung einer Chromatographie zur Abtrennung von mindestens vier Fraktionen kontinuierlich, das heißt unter konstanter Zuführung von Edukten und Entnahme von Produkten.

Bevorzugt wird unter einem kontinuierlichen erfindungsgemäßen Prozess ein Prozess verstanden, bei dem nicht nur das Reaktorsystem kontinuierlich ist, sondern auch die chromatographische Aufreinigung.

Erfindungsgemäß beträgt die zur Durchführung des erfindungsgemäßen Verfahrens verwendete Temperatur in bevorzugter Ausführungsform zu jedem Zeitpunkt höchstens 165 °C, bevorzugt höchstens 160 °C, insbesondere höchstens 150 °C.

Die vorliegende Erfindung ermöglicht die Bereitstellung von Verfahren zur Herstellung von HMF und/oder Ameisensäure und/oder Lävulinsäure, insbesondere zur simultanen Herstellung aus einem Ausgangsmaterial, nämlich einer Fructose-haltigen Ausgangslösung und gegebenenfalls einer rückgeführten Fructose-haltigen Fraktion.

Das erfindungsgemäße Verfahren zur Herstellung von HMF ist in bevorzugter Ausführungsform daher auch ein Verfahren zur Herstellung von HMF und Ameisensäure und Lävulinsäure, welches die Schritte a) bis e), bevorzugt a) bis f), umfasst und der gezielten Herstellung dreier interessierender Produkte dient.

Das erfindungsgemäße Verfahren zur Herstellung von HMF ist in bevorzugter Ausführungsform daher auch ein Verfahren zur Herstellung von HMF und Ameisensäure, welches die Schritte a) bis e), bevorzugt a) bis f), umfasst und das zur Herstellung von zwei interessierenden Wertstoffen dient.

Das erfindungsgemäße Verfahren zur Herstellung von HMF ist in bevorzugter Ausführungsform daher auch ein Verfahren zur Herstellung von HMF und Lävulinsäure, welches die Schritte a) bis e), bevorzugt a) bis f), umfasst und das zur Herstellung von zwei interessierenden Wertstoffen dient.

Bevorzugt werden als Ausgangslösung insbesondere Fructose/Glukose-Sirupe oder Fructose-Sirupe eingesetzt.

In einer bevorzugten Ausführungsform der vorliegenden Erfindung wird vor Schritt b) mindestens eine, vorzugsweise alle, der in Schritt a) bereitgestellten Komponenten auf eine Temperatur von 50 °C bis 165 °C, bevorzugt 60 °C bis 165 °C, bevorzugt 70 °C bis 165 °C, bevorzugt 80 °C bis 165 °C vorgeheizt. In einer bevorzugten Ausführungsform der vorliegenden Erfindung wird vor Schritt b) mindestens eine, vorzugsweise alle, der in Schritt a) bereitgestellten Komponenten auf eine Temperatur von 50 °C bis 160 °C, bevorzugt 60 °C bis 160 °C, bevorzugt 70 °C bis 160 °C, bevorzugt 80 °C bis 160 °C vorgeheizt. In einer bevorzugten Ausführungsform der vorliegenden Erfindung wird vor Schritt b) mindestens eine, vorzugsweise alle, der in Schritt a) bereitgestellten Komponenten auf eine Temperatur von 50 °C bis 150 °C, bevorzugt 60 °C bis 150 °C, bevorzugt 70 °C bis 150 °C, bevorzugt 80 °C bis 150 °C vorgeheizt.

Bevorzugt wird die in Schritt b) erhaltene Reaktionslösung, bevorzugt nach Schritt b) und vor Schritt c), auf eine Temperatur von 50 °C bis 165 °C, bevorzugt 60 °C bis 165 °C, bevorzugt 70 °C bis 165 °C, bevorzugt 80 °C bis 165 °C vorgeheizt. Bevorzugt wird die in Schritt b) erhaltene Reaktionslösung, bevorzugt nach Schritt b) und vor Schritt c), auf eine Temperatur von 50 °C bis 160 °C, bevorzugt 60 °C bis 160 °C, bevorzugt 70 °C bis 160 °C, bevorzugt 80 °C bis 160 °C vorgeheizt. Bevorzugt wird die in Schritt b) erhaltene Reaktionslösung, bevorzugt nach Schritt b) und vor Schritt c), auf eine Temperatur von 50 °C bis 150 °C, bevorzugt 60 °C bis 150 °C, bevorzugt 70 °C bis 150 °C, bevorzugt 80 °C bis 150 °C vorgeheizt.

In einer bevorzugten Ausführungsform der vorliegenden Erfindung werden die in Schritt a) bereitgestellten Komponenten in Schritt b) zum Erhalt einer Reaktionslösung mit einem Kohlenhydratgehalt von 5 Gew.-% bis 50 Gew.-% (Trockensubstanz, im folgenden auch TS, Kohlenhydrat in Bezug auf Gesamtgewicht Reaktionslösung) und einem Fructosegehalt von 40 Gew.-% bis 100 Gew.-% (Trockensubstanz Fructose in Bezug auf Trockensubstanz der Kohlenhydrate) vermischt und anschließend im Reaktor gemäß Verfahrensschritt c) umgesetzt.

In einer weiteren bevorzugten Ausführungsform der vorliegenden Erfindung werden die in Schritt a) bereitgestellte wässrige Fructose-haltigen Ausgangslösung und die gegebenenfalls bereitgestellte wässrige rückgeführte Fructose-haltigen Fraktion vor Schritt b) auf eine Temperatur von 50 °C bis 165 °C, bevorzugt 60 °C bis 165 °C, bevorzugt 70 °C bis 165 °C, bevorzugt 80 °C bis 165 °C, bevorzugt auf eine Temperatur von 50 °C bis 160 °C, bevorzugt 60 °C bis 160 °C, bevorzugt 70 °C bis 160 °C, bevorzugt 80 °C bis 160 °C, bevorzugt auf eine Temperatur von 50 °C bis 150 °C, bevorzugt 60 °C bis 150 °C, bevorzugt 70 °C bis 150 °C, bevorzugt 80 °C bis 150 °C, vorgeheizt und der mindestens eine homogene saure Katalysator, bevorzugt mindestens eine homogene Mineralsäurekatalysator, separat davon vor Schritt b) auf eine Temperatur von 50 °C bis 165 °C, bevorzugt 60 °C bis 165 °C, bevorzugt 70 °C bis 165 °C, bevorzugt 80 °C bis 165 °C, bevorzugt auf eine Temperatur von 50 °C bis 160 °C, bevorzugt 60 °C bis 160 °C, bevorzugt 70 °C bis 160 °C, bevorzugt 80 °C bis 160 °C, bevorzugt auf eine Temperatur von 50 °C bis 150 °C, bevorzugt 60 °C bis 150 °C, bevorzugt 70 °C bis 150 °C, bevorzugt 80 °C bis 150 °C, vorgeheizt und die vorgeheizten Komponenten in Schritt b) zum Erhalt einer Reaktionslösung mit einem Kohlenhydratgehalt von 5 Gew.-% bis 50 Gew.-% (TS Kohlenhydrat in Bezug auf Gesamtgewicht Reaktionslösung) und einem Fructosegehalt von 40 Gew.-% bis 100 Gew.-% (Trockensubstanz Fructose in Bezug auf TS der Kohlenhydrate) vermischt.

In einer weiteren bevorzugten Ausführungsform der vorliegenden Erfindung werden die in Schritt a) bereitgestellte wässrige Fructose-haltige Ausgangslösung und die wässrige rückgeführte Fructose-haltige Fraktion vor Schritt b) auf eine Temperatur von bevorzugt 50 °C bis 165 °C, bevorzugt 60 °C bis 165 °C, bevorzugt 70 °C bis 165 °C, bevorzugt 80 °C bis 165 °C, bevorzugt auf eine Temperatur von 50 °C bis 160 °C, bevorzugt 60 °C bis 160 °C, bevorzugt 70 °C bis 160 °C, bevorzugt 80 °C bis 160 °C, bevorzugt auf eine Temperatur von 50 °C bis 150 °C, bevorzugt 60 °C bis 150 °C, bevorzugt 70 °C bis 150 °C, bevorzugt 80 °C bis 150 °C, vorgeheizt und der mindestens eine homogene saure Katalysator, bevorzugt mindestens eine homogene Mineralsäurekatalysator, mit den vorgeheizten Komponenten in Schritt b) zum Erhalt einer Reaktionslösung mit einem Kohlenhydratgehalt von 5 Gew.-% bis 50 Gew.-% (TS Kohlenhydrate in Bezug auf Gesamtgewicht der Reaktionslösung) und einem Fructosegehalt von 40 Gew.-% bis 100 Gew.-% (Trockensubstanz Fructose in Bezug auf TS der Kohlenhydrate) vermischt.

In einer bevorzugten Ausführungsform der vorliegenden Erfindung beträgt die Konzentration des mindestens einen homogenen sauren Katalysators, bevorzugt mindestens einen homogenen Mineralsäurekatalysators, 0,5 Gew.-% bis 5 Gew.-%, bevorzugt 0,75 Gew.-% bis 3 Gew.-%, bevorzugt 1 Gew.-% bis 2,5 Gew.-% (jeweils Gew.-% in Bezug auf Gesamtgewicht der Reaktionslösung). Bevorzugt liegt die Konzentration des mindestens einen homogenen sauren Katalysators, bevorzugt mindestens einen homogenen Mineralsäurekatalysators, unter 5 Gew.-%, bevorzugt unter 4 Gew.-%, bevorzugt unter 3 Gew.-%, bevorzugt unter 2 Gew.-% (jeweils Gew.-% in Bezug auf Gesamtgewicht der Reaktionslösung). Bevorzugt liegt die Konzentration des mindestens einen homogenen sauren Katalysators, bevorzugt mindestens einen homogenen Mineralsäurekatalysators, über 0,5 Gew.-%, bevorzugt über 0,75 Gew.-%, bevorzugt über 1 Gew.-% (jeweils Gew.-% in Bezug auf Gesamtgewicht der Reaktionslösung).

In einer bevorzugten Ausführungsform ist der mindestens eine homogene saure Katalysator ein homogener Mineralsäurekatalysator.

In einer bevorzugten Ausführungsform der vorliegenden Erfindung ist der mindestens eine homogene saure Katalysator ausgewählt aus Salzsäure (HCl), Schwefelsäure (H₂SO₄), Phosphorsäure (H₃PO₄), aliphatischen oder aromatischen Carbonsäuren und aliphatischen oder aromatischen Sulfonsäuren. In einer bevorzugten Ausführungsform ist der mindestens eine homogene saure Katalysator Schwefelsäure (H₂SO₄). In einer weiteren bevorzugten Ausführungsform ist der mindestens eine homogene saure Katalysator Salzsäure (HCl). In einer weiteren bevorzugten Ausführungsform der vorliegenden Erfindung ist der mindestens eine saure Katalysator Phosphorsäure (H₃PO₄). In einer weiteren bevorzugten Ausführungsform ist der mindestens eine homogene saure Katalysator eine organische Säure, insbesondere eine aliphatische oder aromatische Carbonsäure, zum Beispiel Oxalsäure, Essigsäure oder Benzoesäure oder eine aliphatische oder aromatische Sulfonsäure.

In einer bevorzugten Ausführungsform der vorliegenden Erfindung beträgt der Kohlenhydratgehalt der Reaktionslösung in Schritt b) 5 Gew.-% bis 50 Gew.-%, bevorzugt 5 Gew.-% bis 45 Gew.-%, bevorzugt 7,5 Gew.-% bis 40 Gew.-%, bevorzugt 7,5 Gew.-% bis 35 Gew.-%, bevorzugt 10 Gew.-% bis 30 Gew.-% (jeweils TS in Bezug auf Gesamtgewicht Reaktionslösung).

In einer bevorzugten Ausführungsform beträgt der Fructosegehalt der Reaktionslösung 40 Gew.-% bis 100 Gew.-%, bevorzugt 70 Gew.-% bis 100 Gew.%, bevorzugt 80 Gew.-% bis 100 Gew.%, bevorzugt 90 Gew.-% bis 100 Gew.%, bevorzugt 95 Gew.-% bis 100 Gew.%, bevorzugt 40 Gew.-% bis 99 Gew.-%, bevorzugt 45 Gew.-% bis 99 Gew.-%, bevorzugt 50 Gew.-% bis 95 Gew.-%, bevorzugt 45 Gew.-% bis 90 Gew.-%, bevorzugt 50 Gew.-% bis 85 Gew.-% (jeweils TS Fructose in Bezug auf die Trockensubstanz des Kohlenhydratanteils, das heißt alle in der Reaktionslösung vorhandenen Kohlenhydrate).

In besonders bevorzugter Ausführungsform findet das Vermischen der für die Herstellung der Reaktionslösung eingesetzten Komponenten, das heißt insbesondere der wässrigen Fructose-haltigen Ausgangslösung, gegebenenfalls der wässrigen rückgeführten Fructose-haltigen Fraktion und des mindestens einen homogenen sauren Katalysators in einer Mischvorrichtung und/oder einer Leitung statt. Die Mischvorrichtung und das kontinuierliche Reaktorsystem können räumlich getrennte Baueinheiten darstellen, die durch mindestens eine Leitung miteinander verbunden sind, sie können auch getrennte, aber integrale Bestandteile einer Vorrichtung darstellen.

In einer bevorzugten Ausführungsform der vorliegenden Erfindung erfolgt die Umsetzung der Fructose zu HMF in Schritt c) in einem Zeitraum von 0,1 bis 20 min, insbesondere 0,1 bis 15 min, insbesondere 8 bis 13 min, insbesondere 4 bis 10 min, insbesondere 8 bis 10 min, bevorzugt 0,1 bis 8 min, bevorzugt 0,2 bis 7 min, bevorzugt 0,5 bis 5 min, bevorzugt 1 bis 4 min, bevorzugt 1 bis 3 min. Bevorzugt erfolgt die Umsetzung der Fructose zu HMF in Schritt c) in einem Zeitraum von höchstens 10 min, bevorzugt höchstens 9 min, bevorzugt höchstens 8 min, bevorzugt höchstens 7 min, bevorzugt höchstens 5 min, bevorzugt höchstens 4 min, bevorzugt höchstens 3 min.

In einer besonders bevorzugten Ausführungsform erfolgt die Umsetzung in Schritt c) bei einer Temperatur von 130 bis 150 °C, insbesondere 140 °C für einen Zeitraum von 8 bis 10 min, insbesondere 9 Minuten.

In einer bevorzugten Ausführungsform der vorliegenden Erfindung erfolgt die Umsetzung der Fructose zu HMF in Schritt c) unter Einstellung eines Fructose-Umsatzes von 1 mol-% bis 40 mol-%, bevorzugt 5 mol-% bis 35 mol-%, bevorzugt 10 mol-% bis 30 mol-%, bevorzugt 15 mol-% bis 25 mol-%, bevorzugt 20 mol-% bis 25 mol-%. Bevorzugt erfolgt die Umsetzung der Fructose zu HMF in Schritt c) unter Einstellung eines Fructose-Umsatzes von höchstens 40 mol-%, bevorzugt höchstens 35 mol-%, bevorzugt höchstens 30 mol-%, bevorzugt höchstens 25 mol-%, bevorzugt höchstens 20 mol-%.

In einer bevorzugten Ausführungsform der vorliegenden Erfindung beträgt die HMF-Selektivität in Schritt c) 60 mol-% bis 100 mol-%, bevorzugt 65 mol-% bis 100 mol-%, bevorzugt 70 mol-% bis 100 mol-%, bevorzugt 75 mol-% bis 100 mol-%, bevorzugt 80 mol-% bis 100 mol-%, bevorzugt 85 mol-% bis 100 mol-%, bevorzugt 90 mol-% bis 100 mol-%. Bevorzugt beträgt die HMF-Selektivität in Schritt c) mindestens 60 mol-%, bevorzugt mindestens 65 mol-%, bevorzugt mindestens 70 mol-%, bevorzugt mindestens 75 mol-%, bevorzugt mindestens 80 mol-%, bevorzugt mindestens 85 mol-%, bevorzugt mindestens 90 mol-%, bevorzugt mindestens 95 mol-%.

Im Zusammenhang mit der vorliegenden Erfindung wird die HMF-Selektivität auf den umgesetzten Fructoseanteil bezogen, wobei Anteile anderer Kohlenhydrate, insbesondere Glukose unberücksichtigt bleiben.

In besonders bevorzugter Ausführungsform wird das in Schritt c) eingesetzte kontinuierliche Reaktorsystem als Rohrreaktorsystem ausgeführt. Ein derartiges kontinuierliches Reaktorsystem ist ein dem Fachmann bekanntes Reaktorsystem. In besonders bevorzugter Ausführungsform kann auch ein kontinuierliches Reaktorsystem, insbesondere ein Konti-System, mit geringer Rückvermischung eingesetzt werden. In besonders bevorzugter Ausführungsform kann als kontinuierliches Reaktorsystem ein plug-flow-Reaktor (PFR) eingesetzt werden. In bevorzugter Ausführungsform kann das kontinuierliche Reaktorsystem auch als Strömungsrohr, Rührkessel oder Rührkesselkaskade ausgeführt sein.

In besonders bevorzugter Ausführungsform wird Schritt c) bei einer Temperatur von 80 bis 165 °C, insbesondere 80 bis 160 °C, insbesondere 80 bis 150 °C, insbesondere 85 bis 165 °C, insbesondere 90 bis 160 °C, insbesondere 130 bis 155 °C, insbesondere 135 bis 153 °C, insbesondere 140 bis 150 °C, insbesondere 80 bis 145 °C, insbesondere 100 bis 145 °C, insbesondere 140 °C durchgeführt.

In einer bevorzugten Ausführungsform der vorliegenden Erfindung wird in Schritt c) der Druck zum Umsetzen der in der Reaktionslösung vorhandenen Fructose zu HMF in dem kontinuierlichen Reaktorsystem so eingestellt, dass ein Sieden der Reaktionslösung vermieden wird. Bevorzugt liegt der Druck zum Umsetzen der in der Reaktionslösung vorhandenen Fructose zu HMF in dem kontinuierlichen Reaktorsystem bei 0,1 bis 15 MPa.

Die Erfindung sieht vor, dass in Schritt c) ein Fructoseumsatz von 1 mol-% bis 40 mol-% eingestellt wird. Dies erfolgt erfindungsgemäß bei einer Temperatur von 80 °C bis 165 °C. Bevorzugt ist es erfindungsgemäß möglich, im Rahmen der erfindungsgemäß vorgegebenen Parameter gezielt definierte Fructoseumsätze bereitzustellen, insbesondere durch Verwendung der gegebenen Reaktionstemperatur, gegebenenfalls in bevorzugter Ausführungsform auch der Reaktionszeit, für Schritt c). Anhand dieser Parameter kann auch eine erfindungsgemäß bevorzugte HMF-Selektivität eingestellt werden. In erfindungsgemäß bevorzugter Weise kann die Einstellung des gewünschten Fructoseumsatzes, und gegebenenfalls der HMF-Selektivität, durch Probeentnahme während des Verfahrens, Analyse der Probe und anschließender Berechnung der zum Erreichen der aufrecht zu erhaltenden oder anzupassenden gewünschten Fructoseumsatzwerte und der gegebenenfalls gewünschten HMF-Selektivität erfolgen.

In einer bevorzugten Ausführungsform der vorliegenden Erfindung wird das Produktgemisch in Schritt d) auf eine Temperatur von 20 °C bis 80 °C, bevorzugt 25 °C bis 70 °C, bevorzugt 30 °C bis 60 °C, bevorzugt 30 °C bis 55 °C, bevorzugt 30 °C bis 50 °C, bevorzugt 30 °C bis 45 °C, bevorzugt 30 °C bis 40 °C, bevorzugt genau 80 °C, bevorzugt genau 70 °C, bevorzugt genau 60 °C, bevorzugt genau 55 °C, bevorzugt genau 50 °C, bevorzugt genau 45 °C, bevorzugt genau 40 °C, bevorzugt genau 35 °C, bevorzugt genau 30 °C eingestellt. Bevorzugt wird das Produktgemisch in Schritt d) auf eine Temperatur von höchstens 75°C, bevorzugt höchstens 70 °C, bevorzugt höchstens 60 °C, bevorzugt höchstens 55 °C, bevorzugt höchstens 50 °C, bevorzugt höchstens 45 °C, bevorzugt höchstens 40 °C, bevorzugt höchstens 35 °C eingestellt.

In einer bevorzugten Ausführungsform der vorliegenden Erfindung wird die Temperatur des Produktgemisches in Schritt d) in einem Zeitraum von 0,1 bis 10 min, bevorzugt 0,1 bis 9 min, bevorzugt 0,1 bis 8 min, bevorzugt 0,2 bis 7 min, bevorzugt 0,2 bis 6 min, bevorzugt 0,5 bis 5 min, bevorzugt 0,5 bis 4 min, bevorzugt 0,5 bis 3 min eingestellt. Bevorzugt wird die Temperatur des Produktgemisches in Schritt d) in höchstens 10 min, bevorzugt höchstens 9 min, bevorzugt höchstens 8 min, bevorzugt höchstens 7 min, bevorzugt höchstens 6 min, bevorzugt höchstens 5 min, bevorzugt höchstens 4 min, bevorzugt höchstens 3 min, bevorzugt höchstens 2 min, bevorzugt höchstens 1 min, bevorzugt höchstens 0,5 min eingestellt.

In einer bevorzugten Ausführungsform der vorliegenden Erfindung weist die in Schritt d) erhaltene Produktmischung einen Trockensubstanzgehalt von 5 bis 50 Gew.-%, auf. In einer bevorzugten Ausführungsform der vorliegenden Erfindung weist die in Schritt d) erhaltene Produktmischung einen Trockensubstanzgehalt von 5 bis 70 Gew.-%, auf. In einer bevorzugten Ausführungsform der vorliegenden Erfindung weist die in Schritt d) erhaltene Produktmischung einen Trockensubstanzgehalt von mindestens 5 Gew.-%, auf. In einer bevorzugten Ausführungsform der vorliegenden Erfindung weist die in Schritt d) erhaltene Produktmischung einen Trockensubstanzgehalt von höchstens 70 Gew.-%, auf. In einer bevorzugten Ausführungsform der vorliegenden Erfindung weist die in Schritt d) erhaltene Produktmischung einen Trockensubstanzgehalt von mindestens 10 Gew.-%, auf. In einer bevorzugten Ausführungsform der vorliegenden Erfindung weist die in Schritt d) erhaltene Produktmischung einen Trockensubstanzgehalt von höchstens 60 Gew.-%, auf.In einer weiteren bevorzugten Ausführungsform wird die in Schritt d) erhaltene Produktmischung vor Schritt e) auf einen Trockensubstanzgehalt von 20 bis 50 Gew.-%, bevorzugt 25 bis 50 Gew.-%, bevorzugt 25 bis 45 Gew.-%, bevorzugt 30 bis 45 Gew.-%, bevorzugt 30 bis 40 Gew.-%, aufkonzentriert.

In einer weiteren bevorzugten Ausführungsform wird die in Schritt d) erhaltene Produktmischung vor Schritt e) auf einen Trockensubstanzgehalt von 10 bis 70 Gew.-%, aufkonzentriert. In einer weiteren bevorzugten Ausführungsform wird die in Schritt d) erhaltene Produktmischung vor Schritt e) auf einen Trockensubstanzgehalt von mindestens 5, bevorzugt mindestens 10 Gew.-%, aufkonzentriert. In einer weiteren bevorzugten Ausführungsform wird die in Schritt d) erhaltene Produktmischung vor Schritt e) auf einen Trockensubstanzgehalt von höchstens 70, bevorzugt höchstens 60 Gew.-%, aufkonzentriert.In einer bevorzugten Ausführungsform der vorliegenden Erfindung wird die in Schritt d) erhaltene Produktmischung auf einen Trockensubstanzgehalt von 5 bis 70 Gew.-%, eingestellt. In einer bevorzugten Ausführungsform der vorliegenden Erfindung wird die in Schritt d) erhaltene Produktmischung auf einen Trockensubstanzgehalt von mindestens 5 Gew.-%, eingestellt. In einer bevorzugten Ausführungsform der vorliegenden Erfindung wird die in Schritt d) erhaltene Produktmischung auf einen Trockensubstanzgehalt von höchstens 70 Gew.-%, eingestellt. In einer bevorzugten Ausführungsform der vorliegenden Erfindung wird die in Schritt d) erhaltene Produktmischung auf einen Trockensubstanzgehalt von mindestens 10 Gew.-%, eingestellt. In einer bevorzugten Ausführungsform der vorliegenden Erfindung wird die in Schritt d) erhaltene Produktmischung auf einen Trockensubstanzgehalt von höchstens 60 Gew.-%, eingestellt.

Bevorzugt wird die in Schritt d) erhaltene Produktmischung vor Schritt e) auf einen Wassergehalt von 50 bis 80 Gew.-%, bevorzugt 50 bis 75 Gew.-%, bevorzugt 55 bis 75 Gew.-%, bevorzugt 55 bis 70 Gew.-%, bevorzugt 60 bis 70 Gew.-%, eingestellt.

Bevorzugt wird die in Schritt d) erhaltene Produktmischung vor Schritt e) auf einen Wassergehalt von 30 bis 95 Gew.-%, eingestellt.

Bevorzugt wird die in Schritt d) erhaltene Produktmischung vor Schritt e) auf einen Flüssigkeitsgehalt von 30 bis 95 Gew.-%, eingestellt.

In einer bevorzugten Ausführungsform der vorliegenden Erfindung weist die Schritt e) zugeführte Produktmischung einen Trockensubstanzgehalt von 5 bis 70 Gew.-%, auf. In einer bevorzugten Ausführungsform der vorliegenden Erfindung weist die Schritt e) zugeführte Produktmischung einen Trockensubstanzgehalt von 10 bis 60 Gew.-%, auf. In einer bevorzugten Ausführungsform der vorliegenden Erfindung weist die Schritt e) zugeführte Produktmischung einen Trockensubstanzgehalt von 15 bis 55 Gew.-%, auf.

In einer bevorzugten Ausführungsform der vorliegenden Erfindung ist die Chromatographie eine Chromatographie an Ionenaustauscherharzen, insbesondere an Kationenaustauscherharzen.

In einer besonders bevorzugten Ausführungsform der vorliegenden Erfindung ist die Chromatographie, insbesondere Chromatographie an Ionenaustauscherharzen, insbesondere Chromatographie an Kationenaustauscherharzen, eine Ionenaustauschchromatographie, insbesondere eine Kationenaustauschchromatographie.

Bevorzugt erfolgt die Aufreinigung der in Schritt d) erhaltenen Produktmischung unter Verwendung einer Chromatographie gemäß Schritt e) kontinuierlich. Unter eine kontinuielrichen Chromatographie wird bevorzugt auch eine simulierte Gegenstromchromatographie, wie zum Beispiel die Simulated Moving Bed Chromatography (SMB), verstanden.

Kontinuierliche Chromatographieverfahren sind dem Fachmann allgemein bekannt. Beispielsweise zeigt die US 2011 /0137084 A1 die Funktionsweise der SMB Verfahrens. Weitere geeignete Chroatographieverfahren sind in A. Rajendran et al.; J. Chromatogr. A 1216 (2009), Seiten 709 bis 738 offenbart.

Simulated Moving Bed (SMB) Systeme beziehungsweise Weiterentwicklungen des SMB-Systems, wie zum Beispiel Sequential SMB (SSMB), Intermittent/Improved SMB (ISMB) oder New MCI (NMCI) erlauben in vorteilhafter Weise die Trennung und die Gewinnung der vier beschriebenen Fraktionen in kontinuierlicher Betriebsweise.

In einer bevorzugten Ausführungsform der vorliegenden Erfindung ist die Chromatographie, insbesondere Chromatographie an Ionenaustauscherharzen, in Schritt e) ein Simulated Moving Bed-Verfahren (SMB), ein Sequential Simulated Moving Bed-Verfahren (SSMB) oder ein Improved Simulated Moving Bed-Verfahren beziehungsweise Intermittent Simulated Moving Bed-Verfahren (ISMB). Bevorzugt ist die Chromatographie, insbesondere Chromatographie an Ionenaustauscherharzen, in Schritt e) ein Simulated Moving Bed-Verfahren (SMB), ein Sequential Simulated Moving Bed-Verfahren (SSMB), ein Improved Simulated Moving Bed-Verfahren (ISMB) oder ein New MCI-Verfahren (NMCI). Durch die Verwendung eines Simulated Moving Bed-Verfahrens (SMB), eines Sequential Simulated Moving Bed-Verfahrens (SSMB), eines Improved Simulated Moving Bed-Verfahrens (ISMB) oder eines New MCI-Verfahrens (NMCI) in Schritt e) ist es vorteilhafterweise möglich, die Aufreinigung der in Schritt d) erhaltenen Produktmischung zur Abtrennung einer HMF-Fraktion, einer Kohlcnhydrat/Säurc-Fraktion, einer Fructosc-Fraktion und einer Lävulin- und Ameisensäure-Fraktion in einer kontinuierlichen Fahrweise durchzuführen.

In einer bevorzugten Ausführungsform der vorliegenden Erfindung ist die Chromatographie, insbesondere Chromatographie an Ionenaustauscherharzen, insbesondere an Kationenaustauscherharzen, in Schritt e) ein einstufiges Verfahren. Bevorzugt ist die Chromatographie, insbesondere Chromatographie an Ionenaustauscherharzen, insbesondere an Kationenaustauscherharzen, in Schritt e) ein mehrstufiges Verfahren, bevorzugt ein zweistufiges Verfahren.

Bevorzugt umfasst die Chromatographie, insbesondere Chromatographie an Ionenaustauscherharzen, insbesondere an Kationenaustauscherharzen, in Schritt e) mehrere Stufen, bevorzugt mindestens zwei Stufen, bevorzugt mindestens drei Stufen, bevorzugt genau zwei Stufen, bevorzugt genau drei Stufen.

In einer bevorzugten Ausführungsform der vorliegenden Erfindung kommt es in Schritt e) in einer ersten Stufe der Chromatographie zur Abtrennung von mindestens einer Fraktion, bevorzugt genau einer Fraktion, insbesondere einer HMF-Fraktion oder einer Kohlenhydrat/Säure-Fraktion, bevorzugt von mindestens zwei Fraktionen, bevorzugt genau zwei Fraktionen, bevorzugt genau drei Fraktionen.

In einer weiteren bevorzugten Ausführungsform der vorliegenden Erfindung kommt es in Schritt e) in einer zweiten Stufe der Chromatographie zur Abtrennung von mindestens einer Fraktion, bevorzugt genau einer Fraktion, bevorzugt von mindestens zwei Fraktionen, bevorzugt genau zwei Fraktionen, bevorzugte genau drei Fraktionen, insbesondere einer Kohlenhydrat/Säure-Fraktion, einer Fructose-Fraktion und einer Lävulin- und Ameisensäure-Fraktion oder einer HMF-Fraktion, einer Fructose-Fraktion und einer Lävulin- und Ameisensäure-Fraktion.

In einer bevorzugten Ausführungsform der vorliegenden Erfindung handelt es sich bei der ersten Stufe der Chromatographie in Schritt e) um ein Chromatographieverfahren ausgewählt aus der Gruppe bestehend Simulated Moving Bed-Verfahren (SMB), Sequential Simulated Moving Bed-Verfahren (SSMB), Improved Simulated Moving Bed-Verfahren (ISMB) und New MCI-Verfahren (NMCI).

Bevorzugt handelt es sich bei der ersten Stufe der Chromatographie in Schritt e) um ein Improved Simulated Moving Bed-Verfahren (ISMB). Bevorzugt kommt es in Schritt e) in einer ersten Stufe zur Abtrennung von mindestens einer Fraktion, bevorzugt genau einer Fraktion, insbesondere einer HMF-Fraktion oder einer Kohlenhydrat/Säure-Fraktion, mittels eines Chromatographieverfahrens ausgewählt aus der Gruppe bestehend Simulated Moving Bed-Verfahren (SMB), Sequential Simulated Moving Bed-Verfahren (SSMB), Improved Simulated Moving Bed-Verfahren (ISMB) und New MCI-Verfahren (NMCI), bevorzugt mittels eines Improved Simulated Moving Bed-Verfahren (ISMB).

In einer bevorzugten Ausführungsform der vorliegenden Erfindung handelt es sich bei der zweiten Stufe der Chromatographie in Schritt e) um ein Chromatographieverfahren ausgewählt aus der Gruppe bestehend Simulated Moving Bed-Verfahren (SMB), Sequential Simulated Moving Bed-Verfahren (SSMB), Improved Simulated Moving Bed-Verfahren (ISMB) und New MCI-Verfahren (NMCI).

Bevorzugt handelt es sich bei der ersten Stufe der Chromatographie in Schritt e) um ein New MCI-Verfahren (NMCI). Bevorzugt kommt es in Schritt e) in einer zweiten Stufe zur Abtrennung von mindestens einer Fraktion, bevorzugt genau einer Fraktion, bevorzugt mindestens zwei Fraktionen, bevorzugt genau zwei Fraktionen, bevorzugt mindestens drei Fraktionen, bevorzugt genau drei Fraktionen, insbesondere einer Kohlenhydrat/Säure-Fraktion, einer Fructose-Fraktion und einer Lävulin- und Ameisensäure-Fraktion oder einer HMF-Fraktion, einer Fructose-Fraktion und einer Lävulin- und Ameisensäure-Fraktion, mittels eines Chromatographieverfahrens ausgewählt aus der Gruppe bestehend Simulated Moving Bed-Verfahren (SMB), Sequential Simulated Moving Bed-Verfahren (SSMB), Improved Simulated Moving Bed-Verfahren (ISMB) und New MCI-Verfahren (NMCI), bevorzugt mittels eines New MCI-Verfahrens (NMCI).

Bevorzugt ist insbesondere eine mindestens zweistufige Chromatographie-Auftrennung, bei der in der ersten Stufe die HMF Fraktion abgetrennt wird. Alternativ kann in der ersten Stufe auch die Kohlenhydrat/Säure-Fraktion abgetrennt werden. Bevorzugt ist die erste Stufe der mindestens zweistufigen Chromatographie-Auftrennung ein Moving Bed-Verfahren (ISMB).

Bevorzugt ist die zweite Stufe der mindestens zweistufigen Chromatographie-Auftrennung ein New MCI-Verfahrens (NMCI).

Bevorzugt ist insbesondere eine zweistufige Chromatographie-Auftrennung, bei der in der ersten Stufe die HMF Fraktion abgetrennt wird. Alternativ kann in der ersten Stufe auch die Kohlenhydrat/Säure-Fraktion abgetrennt werden. Bevorzugt ist die erste Stufe der zweistufigen Chromatographie-Auftrennung ein Moving Bed-Verfahren (ISMB). Bevorzugt ist die zweite Stufe der zweistufigen Chromatographie-Auftrennung ein New MCI-Verfahrens (NMCI). Bevorzugt werden in der zweiten Stufe der zweistufigen Chromatographie-Auftrennung die Lävulin-und Ameisensäure-Fraktion, die Fructose-Fraktion und die Kohlenhydrat/Säure-Fraktion voneinander abgetrennt. Alternativ werden in der zweiten Stufe der zweistufigen Chromatographie-Auftrennung die Lävulin-und Ameisensäure-Fraktion, die Fructose-Fraktion und die HMF Fraktion voneinander abgetrennt. In einer bevorzugten Ausführungsform der vorliegenden Erfindung ist die Chromatographie, insbesondere Chromatographie an Ionenaustauscherharzen in Schritt e) eine Chromatographie an Kationenaustauscherharzen.

In einer bevorzugten Ausführungsform der vorliegenden Erfindung wird die Chromatographie, insbesondere Chromatographie an Ionenaustauscherharzen in Schritt e) unter Verwendung eines Kationenaustauscherharzes in H⁺-Form durchgeführt.

In einer weiteren bevorzugten Ausführungsform ist der Chromatographie, insbesondere Chromatographie an Ionenaustauscherharzen, insbesondere an Kationenaustauscherharzen, eine Filtration der Produktmischung, bevorzugt über einen geeigneten Filter oder ein geeignetes Filtersystem, und eine Entfärbung und/oder Reinigung der Produktmischung, bevorzugt eine Entfärbung und/oder Reinigung über Aktivkohle, vorgeschaltet. Bevorzugt erfolgt eine Filtration der Produktmischung über einen geeigneten Filter oder ein geeignetes Filtersystem und eine Entfärbung und/oder Reinigung der Produktmischung zum Beispiel über Aktivkohle nach Schritt d). Bevorzugt erfolgt eine Filtration der Produktmischung über einen geeigneten Filter oder ein geeignetes Filtersystem und eine Entfärbung und/oder Reinigung der Produktmischung zum Beispiel über Aktivkohle vor Schritt e). In besonders bevorzugter Ausführungsform kann nach Verfahrensschritt c), insbesondere nach Verfahrensschritt d), und vor Schritt e) in beliebiger Reihenfolge eine Filtration über einen geeigneten Filter oder ein geeignetes Filtersystem, eine Entfärbung und/oder Reinigung der Produktmischung, insbesondere über Aktivkohle, eine Aufkonzentrierung und gegebenenfalls eine nochmalige Filtration über einen geeigneten Filter oder ein geeignetes Filtersystem durchgeführt werden. In besonders bevorzugter Ausführungsform wird nach Schritt c), insbesondere nach Schritt d), und vor Schritt e) zunächst eine Filtration über einen geeigneten Filter oder ein geeignetes Filtersystem, dann eine Entfärbung und/oder Reinigung, insbesondere über Aktivkohle, dann eine Aufkonzentrierung und gegebenenfalls eine nochmalige Filtration über einen geeigneten Filter oder ein geeignetes Filtersystem in dieser Reihenfolge durchgeführt.

Bevorzugt werden durch die Filtration der Produktmischung über einen geeigneten Filter oder ein geeignetes Filtersystem und Entfärbung und/oder Reinigung über zum Beispiel Aktivkohle unerwünschte Nebenprodukte, insbesondere lösliche und unlösliche Huminstoffe, aus der Produktmischung entfernt. Bevorzugt wird durch die Entfernung von unerwünschten Nebenprodukten, insbesondere von löslichen und unlöslichen Huminstoffen, die Lebensdauer des zur Chromatographie, insbesondere Chromatographie an Ionenaustauscherharzen, insbesondere an Kationenaustauscherharzen, verwendeten Materials, insbesondere Harzes, verlängert.

In einer bevorzugten Ausführungsform wird die Chromatographie, insbesondere Chromatographie an Ionenaustauscherharzen, in Schritt e) bei einer Temperatur von 40 °C bis 80 °C, bevorzugt 40 °C bis 70 °C, bevorzugt 40 °C bis 60 °C, bevorzugt 50 °C bis 80 °C, bevorzugt 50 °C bis 70 °C, bevorzugt 50 °C bis 60 °C, bevorzugt 60 °C bis 80 °C, bevorzugt 60 °C bis 70 °C, durchgeführt.

Die in Schritt e) aufgetrennte, bevorzugt in einem Schritt f) erhaltene, Fructose-Fraktion wird bevorzugt kontinuierlich in den Verfahrensschritt a) rückgeführt. Dabei ist die in Schritt e) aufgetrennte, bevorzugt in einem Schritt f) erhaltene, Fructose-Fraktion vorteilhafterweise weitestgehend, bevorzugt vollständig, von gebildeter Lävulinsäure befreit. In einer weiteren bevorzugten Ausführungsform ist die in Schritt e) aufgetrennte, bevorzugt in einem Schritt f) erhaltene, Fructose-Fraktion vorteilhafterweise weitestgehend, bevorzugt vollständig, von gebildeter Lävulin- und Ameisensäure befreit.

In einer besonders bevorzugten Ausführungsform wird die in Schritt e) aufgetrennte, bevorzugt in einem Schritt f) erhaltene, Fructose-Fraktion, gegebenenfalls nach Aufkonzentrierung, kontinuierlich vollständig in den Schritt a) rückgeführt. In einer weiteren bevorzugten Ausführungsform wird die in Schritt e) aufgetrennte, bevorzugt in einem Schritt f) erhaltene, Fructose-Fraktion kontinuierlich, gegebenenfalls nach Aufkonzentrierung, zumindest teilweise, insbesondere zu mindestens 70 %, bevorzugt zu mindestens 80 %, bevorzugt zu mindestens 90 %, bevorzugt zu mindestens 95 %, bevorzugt zu mindestens 98 %, bevorzugt zu mindestens 99 %, in Schritt a) rückgeführt (jeweils Gew.-% der zurückgeführten Fructose-Fraktion in Bezug auf die in Schritt e) aufgetrennte, bevorzugt in einem Schritt f) erhaltene, Fructose-Fraktion).

In besonders bevorzugter Ausführungsform ist das Verhältnis von Fructose zu Glukose in der rückgeführten Fructose-Fraktion nicht kleiner als in der in Schritt a) bereitgestellten wässrigen Fructose-haltigen Ausgangslösung.

In einer weiteren bevorzugten Ausführungsform der vorliegenden Erfindung weist die in Schritt e) aufgetrennte, bevorzugt in einem Schritt f) erhaltene, Kohlenhydrat/Säure-Fraktion eine hinreichend hohe Reinheit auf, ist insbesondere frei von Fermentationsinhibitoren, dass sie direkt, gegebenenfalls nach Aufkonzentrierung, sowohl als Feed (Einspeisungsmaterial) in fermentativen Prozessen, insbesondere zur Ethanolherstellung, insbesondere Ethanol-Fermentation, als auch als Edukt in chemischen Prozessen, insbesondere der Oxidation von Glukose zu Gluconsäure, eingesetzt werden kann.

In einer weiteren bevorzugten Ausführungsform wird die in Schritt e) aufgetrennte, bevorzugt in einem Schritt f) erhaltene, Kohlenhydrat/Säure-Fraktion für die Ethanolherstellung, insbesondere Ethanol-Fermentation, insbesondere für die Bio-Ethanol-Gewinnung, und zur Gluconsäure-Gewinnung eingesetzt.

Die vorliegende Erfindung stellt daher auch ein Verfahren zur Herstellung eines Feeds für fermentative Prozesse, insbesondere zur Ethanolherstellung, insbesondere Ethanol-Fermentation, oder zur Herstellung eines Ausgangsmaterials, das heißt Eduktes, in chemischen Prozessen, insbesondere zur Herstellung von Gluconsäure, bereit, im Rahmen dessen ein Verfahren der vorliegenden Erfindung mit den Verfahrensschritten a) bis e), bevorzugt a) bis f), unter Erhalt einer Kohlenhydrat/Säure-Fraktion, die als Feed oder Edukt eingesetzt werden kann, durchgeführt wird.

In einer bevorzugten Ausführungsform der vorliegenden Erfindung wird die in Schritt e) aufgetrennte, bevorzugt in einem Schritt f) erhaltene, Kohlenhydrat/Säure-Fraktion für die Ethanolherstellung, insbesondere für die Ethanol-Fermentation, insbesondere für die Bio-Ethanol-Gewinnung, eingesetzt.

In einer besonders bevorzugten Ausführungsform wird ein Verfahren für die Ethanolherstellung, insbesondere Ethanol-Fermentation bereitgestellt, im Rahmen dessen das erfindungsgemäße Verfahren, insbesondere die Verfahrensschritte a) bis e), bevorzugt a) bis f), insbesondere zum Erhalt einer Kohlenhydrat/Säure-Fraktion durchgeführt werden, wobei die erhaltene Kohlcnhydrat/Säurc-Fraktion für die Ethanolherstellung, insbesondere Ethanol-Fcrmcntation, insbesondere für die Bio-Ethanol-Gewinnung, eingesetzt wird.

In einer weiteren bevorzugten Ausführungsform wird im Falle der Verwendung von Schwefelsäure als homogener saurer Katalysator, bevorzugt Mineralsäurekatalysator, in dem erfindungsgemäßen Verfahren die Schwefelsäure in Schritt e) als Schwefelsäure-Fraktion abgetrennt und die in einem Schritt f) erhaltene Schwefelsäure-Fraktion für die Ethanolherstellung, insbesondere Ethanol-Fermentation, insbesondere für die Bio-Ethanol-Gewinnung, eingesetzt.

In einer weiteren bevorzugten Ausführungsform wird die in Schritt e) aufgetrennte, bevorzugt in einem Schritt f) erhaltene, Kohlenhydrat/Säure-Fraktion zur Gluconsäure-Gewinnung eingesetzt, gegebenenfalls nach Aufkonzentrierung.

In einer besonders bevorzugten Ausführungsform wird ein Verfahren zur Herstellung von Gluconsäure bereitgestellt, dass das erfindungsgemäße Verfahren umfasst, insbesondere die Verfahrensschritte a) bis e), bevorzugt a) bis f), insbesondere zum Erhalt einer Kohlenhydrat/Säure-Fraktion, die zur Gewinnung von Glukose und zur anschließenden Oxidation der Glukose zu Gluconsäure eingesetzt wird.

In einer bevorzugten Ausführungsform der vorliegenden Erfindung wird die in Schritt e) aufgetrennte, bevorzugt in einem Schritt f) erhaltene, Lävulin- und Ameisensäure-Fraktion zur Isolierung von Lävulin- und Ameisensäure eingesetzt. In einer weiteren bevorzugten Ausführungsform wird die in Schritt e) aufgetrennte, bevorzugt in einem Schritt f) erhaltene, Lävulin- und Ameisensäure-Fraktion zur Isolierung von Lävulinsäure eingesetzt. In einer weiteren bevorzugten Ausführungsform wird die in Schritt e) aufgetrennte, bevorzugt in einem Schritt f) erhaltene, Lävulin- und Ameisensäure-Fraktion zur Isolierung von Ameisensäure eingesetzt.

Die vorliegende Erfindung betrifft daher auch ein Verfahren zur Herstellung von Lävulinsäure, Ameisensäure oder Lävulinsäure und Ameisensäure, wobei ein Verfahren, umfassend die Schritte a) bis e), bevorzugt a) bis f), der vorliegenden Erfindung durchgeführt wird und in einem Schritt f) Lävulinsäure, Ameisensäure oder Lävulinsäure und Ameisensäure erhalten wird.

In einer weiteren bevorzugten Ausführungsform der vorliegenden Erfindung wird die in Schritt e) aufgetrennte, bevorzugt in einem Schritt f) erhaltene, HMF-Fraktion direkt, gegebenenfalls nach Aufkonzentrierung, das heißt ohne die Notwendigkeit aufwendiger weiterer Aufreinigung, in einem zusätzlichen Schritt zu 2,5-Furandicarbonsäure (FDCA) oxidiert.

Die vorliegende Erfindung betrifft daher auch ein Verfahren zur Herstellung von FDCA, umfassend die Schritte a) bis e), bevorzugt a) bis f), der vorliegenden Erfindung, wobei die in Schritt e) aufgetrennte, bevorzugt in einem Schritt f) erhaltene, HMF-Fraktion, vorzugsweise direkt, gegebenenfalls nach Aufkonzentrierung, und ohne die Notwendigkeit aufwendiger weiterer Aufreinigung, zu FDCA oxidiert wird.

Erfindungsgemäß umfasst die in Schritt e) aufgetrennte, bevorzugt in einem Schritt f) erhaltene, Kohlenhydrat/Säure-Fraktion mindestens 20 Gew.-% der in der Produktmischung enthaltenen Glukose (jeweils TS bezogen auf Produktmischung).

Erfindungsgemäß enthält die Kohlenhydrat/Säure-Fraktion 0,8 Gew.-% bis 100 Gew.-% Glukose, 0 Gew.-% bis 99,2 Gew.-% Fructose, höchstens 2 Gew.-%, bevorzugt höchstens 1 Gew.-%, bevorzugt höchstens 0,5 Gew.-%, bevorzugt höchstens 0,1 Gew.-%, Lävulin- und Ameisensäure und höchstens 10 Gew.-%, bevorzugt höchstens 5 Gew.-%, bevorzugt höchstens 2 Gew.-%, mehr bevorzugt höchstens 1 Gew.-%, bevorzugt höchstens 0,5 Gew.-%, bevorzugt höchstens 0,1 Gew.-%, HMF (jeweils TS, bezogen auf Summe der analysierten Komponenten (Glukose, Fructose, Lävulinsäure, Ameisensäure, HMF, Difructoseanhydride (DFA))). Erfindungsgemäß bevorzugt enthält die Kohlenhydrat/Säure-Fraktion höchstens 10 Gew.-%, mehr bevorzugt höchstens 5 Gew.-% HMF.

Die in Schritt e) aufgetrennte, bevorzugt in einem Schritt f) erhaltene, Fructose-Fraktion enthält erfindungsgemäß mindestens 70 Gew.-%, bevorzugt mindestens 80 Gew.-%, der in der Produktmischung enthaltenen Fructose (jeweils TS bezogen auf Produktmischung).

Erfindungsgemäß enthält die Fructose-Fraktion 0 Gew.-% bis 60 Gew.-% Glukose, 40 Gew.-% bis 100 Gew.-% Fructose, höchstens 2 Gew.-%, bevorzugt höchstens 1 Gew.-%, bevorzugt höchstens 0,5 Gew.-%, bevorzugt höchstens 0,1 Gew.-% Lävulinsäure, höchstens 2 Gew.-%, bevorzugt höchstens 1,5 Gew.-%, bevorzugt höchstens 1 Gew.-%, bevorzugt höchstens 0,5 Gew.-%, bevorzugt höchstens 0,25 Gew.-%, bevorzugt höchstens 0,1 Gew.-%, Ameisensäure und höchstens 2 Gew.-%, bevorzugt höchstens 1,5 Gew.-%, bevorzugt höchstens 1 Gew.-%, bevorzugt höchstens 0,8 Gew.-%, bevorzugt höchstens 0,6 Gew.-%, bevorzugt höchstens 0,4 Gew.-%, bevorzugt höchstens 0,2 Gew.-%, bevorzugt höchstens 0,1 Gew.-%, HMF (jeweils TS, bezogen auf Summe der analysierten Komponenten (Glukose, Fructose, Lävulinsäure, Ameisensäure, HMF, Difructoseanhydride (DFA)). Erfindungsgemäß bevorzugt enthält die Fructose-Fraktion höchstens 2 Gew.-%, HMF. Erfindungsgemäß bevorzugt enthält die Fructose-Fraktion höchstens 2 Gew.-%, Lävulinsäure. In besonders bevorzugter Ausführungsform ist das Verhältnis von Fructose zu Glukose in der Fructose-Fraktion nicht kleiner als in der in Schritt a) bereitgestellten wässrigen Fructose-haltigen Ausgangslösung.

Erfindungsgemäß enthält die in Schritt e) aufgetrennte, bevorzugt in einem Schritt f) erhaltene, Lävulin- und Ameisensäure-Fraktion mindestens 60 Gew.-%, bevorzugt mindestens 65 Gew.-%, bevorzugt mindestens 70 Gew.-%, bevorzugt mindestens 80 Gew.-%, bevorzugt mindestens 90 Gew.-%, bevorzugt mindestens 95 Gew.-%, bevorzugt mindestens 98 Gew.-%, bevorzugt mindestens 99 Gew.-%, bevorzugt mindestens 99,5 Gew.-%, bevorzugt mindestens 99,8 Gew.-%, bevorzugt 100 Gew.-% der in der Produktmischung enthaltenen Lävulin- und Ameisensäure (jeweils TS, bezogen auf Produktmischung).

Erfindungsgemäß enthält die Lävulin- und Ameisensäure-Fraktion 50 Gew.-% bis 100 Gew.-%, bevorzugt 60 Gew.-% bis 100 Gew.-%, bevorzugt, mehr bevorzugt 65 Gew.-% bis 100 Gew.-%, bevorzugt 70 Gew.-% bis 100 Gew.-%, bevorzugt 80 Gew.-% bis 100 Gew.-%, bevorzugt 90 Gew.-% bis 100 Gew.-%, bevorzugt 95 Gew.-% bis 100 Gew.-%, bevorzugt 98 Gew.-% bis 100 Gew.-%, bevorzugt 99 Gew.-% bis 100 Gew.-%, bevorzugt 99,5 Gew.-% bis 100 Gew.-%, bevorzugt 99,7 Gew.-% bis 100 Gew.-% Lävulin- und Ameisensäure (jeweils TS, bezogen auf Summe der analysierten Komponenten (Glukose, Fructose, Lävulinsäure, Ameisensäure, HMF, Difructoseanhydride (DFA))). Erfindungsgemäß bevorzugt enthält die Lävulin- und Ameisensäure-Fraktion mindestens 50 Gew.-% Lävulinsäure, mehr bevorzugt mindestens 60 Gew.-% Lävulinsäure, mehr bevorzugt mindestens 70 Gew.-% Lävulinsäure.

Die in Schritt e) aufgetrennte, bevorzugt in einem Schritt f) erhaltene, HMF-Fraktion enthält erfindungsgemäß mindestens 70 Gew.-%, bevorzugt mindestens 80 Gew.-%, mehr bevorzugt mindestens 90 Gew.-%, bevorzugt mindestens 98 Gew.-%, bevorzugt mindestens 99 Gew.-%, bevorzugt mindestens 99,5 Gew.-%, bevorzugt mindestens 99,8 Gew.-%, bevorzugt 100 Gew.-% des in der Produktmischung enthaltenen HMF (jeweils TS, bezogen auf Produktmischung).

Erfindungsgemäß enthält die HMF-Fraktion 80 Gew.-% bis 100 Gew.-%, bevorzugt 85 Gew.-% bis 100 Gew.-%, bevorzugt 90 Gew.-% bis 100 Gew.-%, bevorzugt 95 Gew.-% bis 100 Gew.-%, bevorzugt 98 Gew.-% bis 100 Gew.-%, bevorzugt 99 Gew.-% bis 100 Gew.-%, bevorzugt 99,5 Gew.-% bis 100 Gew.-%, bevorzugt 99,7 Gew.-% bis 100 Gew.-% HMF und höchstens 16 Gew.-%, bevorzugt höchstens 14 Gew.-%, bevorzugt höchstens 12 Gew.-%, bevorzugt höchstens 10 Gew.-%, bevorzugt höchstens 8 Gew.-%, bevorzugt höchstens 6 Gew.-%, bevorzugt höchstens 4 Gew.-%, bevorzugt höchstens 2 Gew.-%, bevorzugt höchstens 1 Gew.-%, Lävulin- und Ameisensäure, höchstens 2 Gew.-%, bevorzugt höchstens 1 Gew.-%, bevorzugt höchstens 0,8 Gew.-%, bevorzugt höchstens 0,6 Gew.-%, bevorzugt höchstens 0,4 Gew.-%, bevorzugt höchstens 0,2 Gew.-%, bevorzugt höchstens 0,1 Gew.-%, Glukose und höchstens 2 Gew.-%, bevorzugt höchstens 1 Gew.-%, bevorzugt höchstens 0,8 Gew.-%, bevorzugt höchstens 0,6 Gew.-%, bevorzugt höchstens 0,4 Gew.-%, bevorzugt höchstens 0,2 Gew.-%, bevorzugt höchstens 0,1 Gew.-%, Fructose (jeweils TS, bezogen auf Summe der analysierten Komponenten (Glukose, Fructose, Lävulinsäure, Ameisensäure, HMF, Difructoseanhydride (DFA))).

In einer bevorzugten Ausführungsform werden in dem erfindungsgemäßen Verfahren, insbesondere während der Schritte a) bis e), bevorzugt a) bis f), keine organischen Lösungsmittel eingesetzt.

In einer bevorzugten Ausführungsform wird das erfindungsgemäße Verfahren, insbesondere während der Schritte a) bis e), bevorzugt a) bis f), nicht unter sauerstoffreduzierten Bedingungen durchgeführt.

In einer bevorzugten Ausführungsform wird die Reaktionslösung nicht mittels Dampfinjektion auf eine Temperatur von 80 bis 165 °C gebracht.

Im Zusammenhang mit der vorliegenden Erfindung wird unter einem plug-flow-Reaktor (PFR) ein sogenanntes ideales Strömungsrohr (IR) verstanden, das heißt ein rohrförmiger Reaktor, in welchem eine Tropfenströmung vorliegt. Ein derartiger Reaktor ist insbesondere auch dadurch ausgezeichnet, dass keine Vermischung, Rückströmung oder Verwirbelung der durchgeführten Reaktionslösung stattfindet, sondern vielmehr eine gleichmäßige Durchströmung unter parallel stattfindender Stoffumwandlung stattfindet. Der plug-flow-Reaktor stellt insbesondere sicher, dass jeder in den plug-flow-Reaktor eingespeiste Stoff, insbesondere jede eingespeiste Komponente, unter gleichen Bedingungen kontinuierlich umgesetzt wird, das heißt alle Komponenten dem Umsetzungsprozess für die gleiche Zeitdauer ausgesetzt werden.

Erfindungsgemäß wird unter einer "wässrigen rückgeführten Fructose-haltigen Fraktion" eine aus der gemäß dem erfindungsgemäßen Verfahren durchgeführten Chromatographie, insbesondere Chromatographie an Ionenaustauscherharzen, insbesondere an Kationenaustauscherharzen, erhaltene wässrige Fraktion nicht umgesetzter Fructose verstanden, die weitestgehend, bevorzugt vollständig, von während der Fructoseumsetzung gebildeten Nebenprodukten, insbesondere Lävulin- und Ameisensäure und Huminstoffen, befreit ist. Dabei weist die erhaltene wässrige Fraktion nicht umgesetzter Fructose eine derart hohe Reinheit auf, dass sie in einer bevorzugten Ausführungsform direkt, gegebenenfalls nach Aufkonzentrierung, das heißt ohne weitere Aufreinigung, in den Verfahrensschritt a) zurückgeführt wird und nach dem Vermischen mit der wässrigen Fructose-haltigen Ausgangslösung und dem mindestens einen homogenen sauren Katalysator für eine weitere Umsetzung zu HMF zur Verfügung steht. Da in dieser bevorzugten Ausführungsform bei der Initiierung des erfindungsgemäßen Verfahrens zunächst noch keine wässrige rückgeführte Fructose-haltige Fraktion zur Verfügung steht, wird in diesem Fall stattdessen bevorzugt eine entsprechend größere Menge an wässriger Fructose-haltiger Ausgangslösung verwendet.

Im Zusammenhang mit der vorliegenden Erfindung wird unter "Einstellung eines Fructose-Umsatzes" verstanden, dass die für die Umsetzung von Fructose zu HMF verwendeten Reaktionsparameter, insbesondere die Reaktionstemperatur und die Reaktionsdauer im Reaktor so gewählt werden, dass es lediglich zu einer limitierten Umsetzung der Fructose von maximal 40 mol-% kommt, wodurch eine hohe HMF-Selektivität und damit verbunden eine geringe Nebenproduktbildung erreicht werden kann.

Unter dem Begriff "und/oder" wird in Zusammenhang mit der vorliegenden Erfindung verstanden, dass alle Mitglieder einer Gruppe, welche durch den Begriff "und/oder" verbunden sind, sowohl alternativ zueinander als auch jeweils untereinander kumulativ in einer beliebigen Kombination offenbart sind. Dies bedeutet für den Ausdruck "A, B und/oder C", dass folgender Offenbarungsgehalt darunter zu verstehen ist: A oder B oder C oder (A und B) oder (A und C) oder (B und C) oder (A und B und C).

Im Zusammenhang mit der vorliegenden Erfindung wird unter dem Begriff "umfassend" verstanden, dass zusätzlich zu den von dem Begriff explizit erfassten Elementen noch weitere, nicht explizit genannte Elemente hinzutreten können. Im Zusammenhang mit der vorliegenden Erfindung wird unter diesem Begriffen auch verstanden, dass allein die explizit genannten Elemente erfasst werden und keine weiteren Elemente vorliegen. In dieser besonderen Ausführungsform ist die Bedeutung des Begriffes "umfassend" gleichbedeutend mit dem Begriff "bestehend aus". Darüber hinaus erfasst der Begriff "umfassend" auch Gesamtheiten, die neben den explizit genannten Elementen auch weitere nicht genannte Elemente enthalten, die sich jedoch von funktionell und qualitativ untergeordneter Natur sind. In dieser Ausführungsform ist der Begriff "umfassend" gleichbedeutend mit dem Begriff "im Wesentlichen bestehend aus".

Weitere bevorzugte Ausführungsformen ergeben sich aus den Unteransprüchen.

Die vorliegende Erfindung wird anhand des nachfolgenden Beispiels und dazugehörigen Figuren illustriert.
Figur 1 zeigt ein Prozessschema des erfindungsgemäßen Verfahrens mit getrenntem Vorheizen von Kohlenhydratlösung und saurem Katalysator vor dem Mischen der Komponenten.
Figur 2 zeigt ein Prozessschema des erfindungsgemäßen Verfahrens mit gemeinsamem Vorheizen der Komponenten nach dem Mischen der Komponenten.

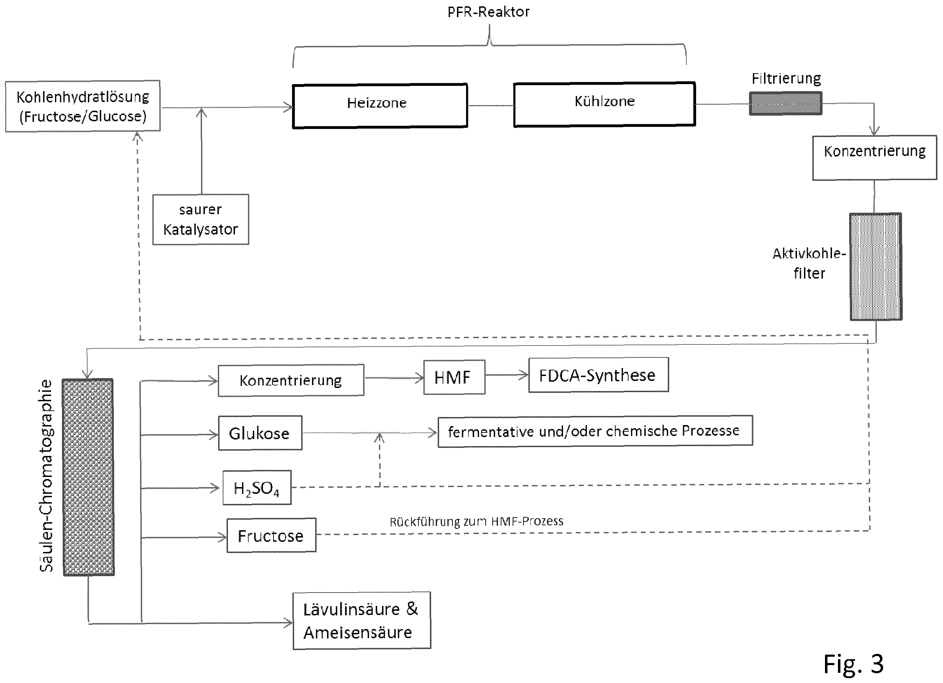

Figur 3 zeigt ein Prozessschema des erfindungsgemäßen Verfahrens mit Mischen von Kohlenhydratlösung und saurem Katalysator vor dem Reaktor ohne vorheriges Aufheizen.
Figur 4 zeigt den Reaktionsverlauf zu Beispiel 1, 18,5 Gew.-% Kohlenhydratlösung zurückgeführt aus Chromatographie, 0,75 Gew.-% Schwefelsäure, 150 °C, VWZ (Verweilzeit, also Umsetzungszeit gemäß Verfahrensschritt c)) 6 min. Dabei ist die Kohlenstoffbilanz (6), HMF-Selektivität (7), Fructose-Umsatz (8), Ameisensäure (9), Lävulinsäure (10).
Figur 5 zeigt den Reaktionsverlauf zu Beispiel 2; 20 Gew.-% Kohlenhydrat, 0,75 Gew.-% Schwefelsäure, 150 °C, VWZ 6 min. Dabei ist die Kohlenstoffbilanz (6), HMF-Selektivität (7), Fructose-Umsatz (8), Ameisensäure (9), Lävulinsäure (10).
Figur 6 zeigt den Reaktionsverlauf zu Beispiel 3; 15 Gew.-% Kohlenhydrat, 0,5 Gew.-% Schwefelsäure, 153 °C, VWZ 7 min. Dabei ist die Kohlenstoffbilanz (6), HMF-Selektivität (7), Fructose-Umsatz (8), Ameisensäure (9), Lävulinsäure (10).
Figur 7 zeigt den Reaktionsverlauf zu Beispiel 4; 25 Gew.-% Kohlenhydrat, 0,75 Gew.-% Schwefelsäure, 150 °C, VWZ 6 min. Dabei ist die Kohlenstoffbilanz (6), HMF-Selektivität (7), Fructose-Umsatz (8), Ameisensäure (9), Lävulinsäure (10).
Figur 8 zeigt den Reaktionsverlauf zu Beispiel 5; 15 Gew.-% Kohlenhydrat (ab 23 h erhöht auf 20 Gew.-% Kohlenhydrat), 1,0 % Schwefelsäure, 140 °C, VWZ 9 min. Dabei ist die Kohlenstoffbilanz (6), HMF-Selektivität (7), Fructose-Umsatz (8), Ameisensäure (9), Lävulinsäure (10).
Figur 9 zeigt die Ergebnisse einer Oxidation einer HMF-Lösung aus dem erfindungsgemäßen Prozess. HMF = 5-Hydroxymethylfurfural (25), HMCA = 5-Hydroxymethyl-2-furancarbonsäure (29), FFCA = 5-Formyl-2-furancarbonsäure (26), FDCA = 2,5-Furandicarbonsäure (27), FDC = 2,5-Furandicarboxaldehyd (28), Umsatz aus NaOH-Kurve (30).
Figur 10 zeigt schematisch die Durchführung einer zweistufigen chromatographischen Auftrennung eines HMF-haltigen Produktgemisches mit niedrigem Trockensubstanzanteil in eine HMF-Fraktion (Extrakt), eine Fructose-Fraktion (P-Fraktion), eine Lävulin- und Ameisensäure-Fraktion (Q-Fraktion) und eine Kohlenhydrat/Säure-Fraktion (R-Fraktion). ISMB = Improved Simulated Moving Bed, NMCI = New MCI.
Figur 11 zeigt schematisch die Durchführung einer zweistufigen chromatographischen Auftrennung eines HMF-haltigen Produktgemisches mit niedrigem Trockensubstanzanteil in eine Kohlenhydrat/Säure-Fraktion (Raffinat), eine Lävulin- und Ameisensäure-Fraktion (P-Fraktion), eine HMF-Fraktion (Q-Fraktion) und eine Fructose-Fraktion (R-Fraktion). ISMB = Improved Simulated Moving Bed, NMCI = New MCI.
Figur 12 zeigt schematisch die Durchführung einer zweistufigen chromatographischen Auftrennung eines HMF-haltigen Produktgemisches mit hohem Trockensubstanzanteil in eine HMF-Fraktion (Extrakt), eine Fructose-Fraktion (P-Fraktion), eine Lävulin- und Ameisensäure-Fraktion (Q-Fraktion) und eine Kohlenhydrat/Säure-Fraktion (R-Fraktion). ISMB = Improved Simulated Moving Bed, NMCI = New MCI.
Figur 13 zeigt schematisch die Durchführung einer zweistufigen chromatographischen Auftrennung eines HMF-haltigen Produktgemisches mit hohem Trockensubstanzanteil in eine Kohlenhydrat/Säure-Fraktion (Raffinat), eine Lävulin- und Ameisensäure-Fraktion (P-Fraktion), eine HMF-Fraktion (Q-Fraktion) und eine Fructose-Fraktion (R-Fraktion). ISMB = Improved Simulatcd Moving Bed, NMCI = New MCI.

### Beispiele:

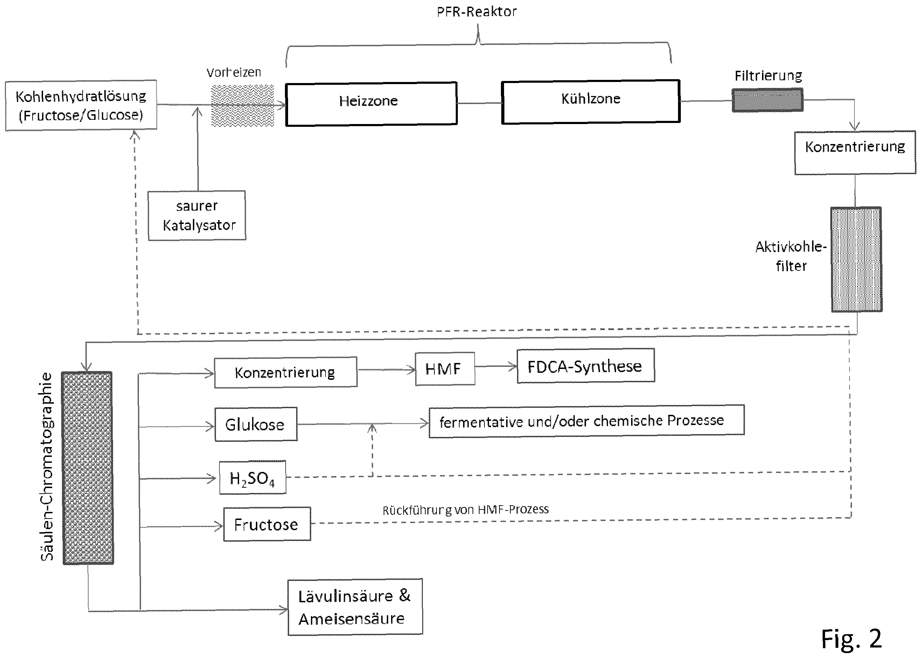

### A) Allgemeiner Versuchsaufbau gemäß Figur 2

Als Reaktionslösung dient eine Kohlenhydratlösung mit variablem Fructose/Glukoseverhältnis in 0,5 - 1,0 Gew.-% Schwefelsäure. Der Kohlenhydratanteil beträgt 15% bis 25% Trockensubstanz (TS, bezogen auf Gesamtgewicht Reaktionslösung).

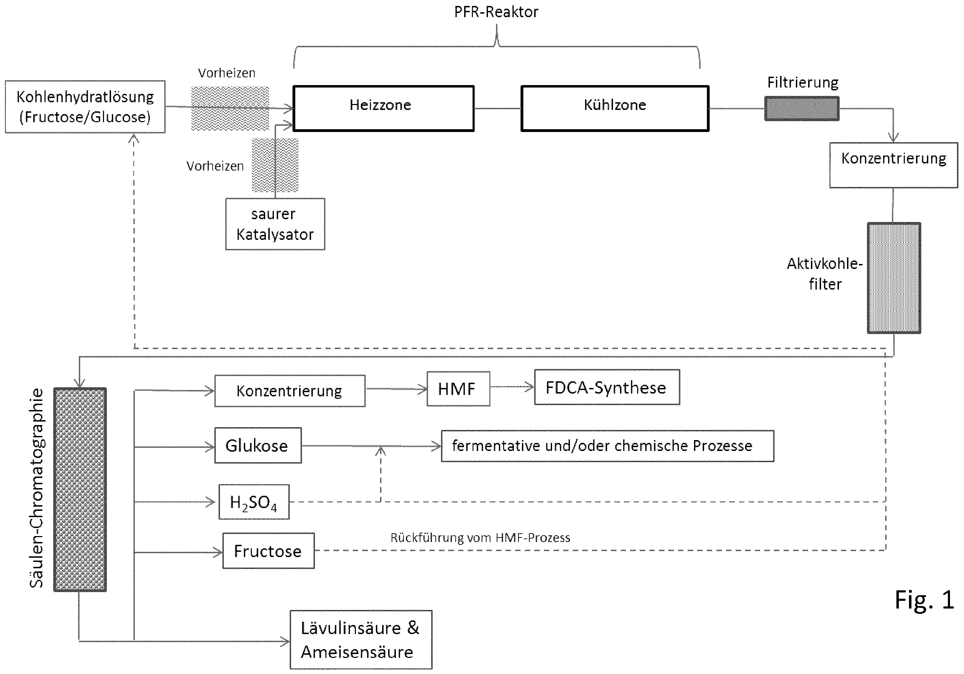

Die Reaktionslösung wird mit Hilfe einer HPLC-Pumpe zunächst durch einen Doppelrohrwärmetauscher (Innendurchmesser 3 mm, Länge 1614 mm, Volumen 11,4 ml) und anschließend in den kontinuierlich betriebenen Rohrreaktor (Innendurchmesser 6 mm, Länge 630 mm, Volumen 17,8 ml) gepumpt (Figur 2, alternative Ausführung mit getrenntem Vorheizen gemäß Figur 1). Die Temperierung des Doppelrohrwärmetauschers erfolgt dabei mit einem mit Ethylenglycol als Heizmedium betriebenen Thermostaten. Die Temperierung des eigentlichen Reaktors erfolgt mittels einer Elektroheizung in Form eines Heizschlauchs. Der Temperaturfühler für die Regelung des Heizschlauches befindet sich dabei im Inneren des Heizschlauchs etwa auf halber Höhe. Kontrollmessungen zeigten, dass im verwendeten Verweilzeitbereich eine maximale Temperaturdifferenz zwischen dem Temperaturfühler im Heizschlauch und der Temperatur der Reaktionslösung am Reaktor besteht (gemessen in der Reaktionslösung in der Rohrmitte). Angegeben ist jeweils die Regeltemperatur des Heizschlauchs. Nach der Heizzone des Reaktors erfolgt direkt der Übergang in die Kühlzone (Doppelrohrwärmetauscher mit Innendurchmesser 6 mm, Länge 400 mm, Volumen 11,3 ml). Innerhalb der Kühlzone wird die Produktmischung von Reaktionstemperatur auf ca. 50 °C abgekühlt, im Anschluss daran wird die Lösung über einen Sinterfilter (7 µm Porenweite) filtriert und gesammelt. Der Druck im Reaktionssystem wird mit Hilfe eines Druckhalteventils so eingestellt, dass ein Sieden der Reaktionslösung (und damit ein Ausgasen) vermieden wird. (ca. 0,5- 0,6 MPa bei einer Reaktionstemperatur von 150 °C).

### B) Beispiel 1: HMF-Synthese mit einer aus der Chromatographie zurückgeführten Fructoselösung (18,5 Gew.-%) bei 150 °C in 0,75 % Säure

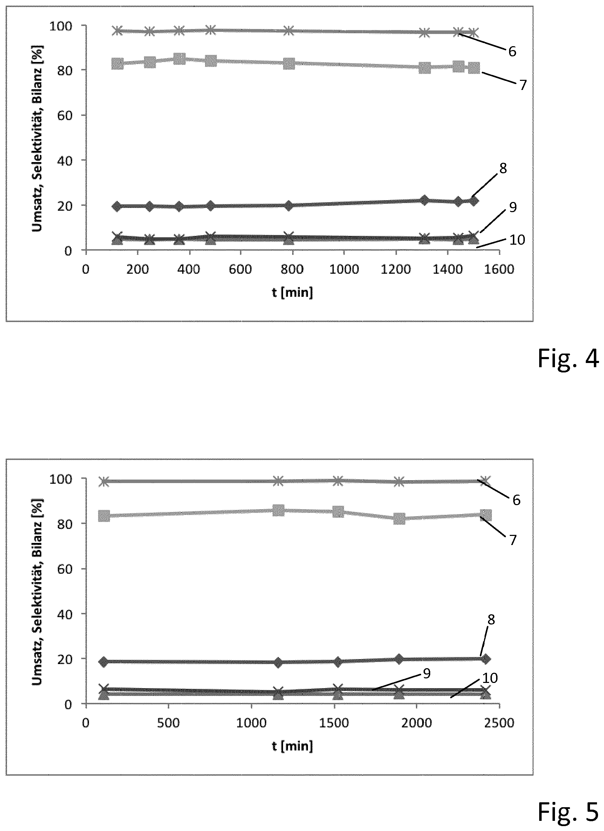

Eine 18,5 Gew.-% Kohlenhydratlösung (gemäß A, vorstehend) (86,1% Fructose, 13,8 % Glukose, 0,75 Gew.-% Schwefelsäure), bei der 100 % des Kohlenhydratanteils aus einer aus dem chromatographischen Trennprozess rückgeführter Fructose-Fraktion besteht, wurde zunächst auf 80 °C vorgeheizt und dann in Schritt c) bei einer Temperatur von 150 °C mit einer Verweilzeit (bezogen auf die Heizzone) von 6 min über eine Versuchsdauer von 25 h kontinuierlich umgesetzt. Im Reaktionsverlauf wurden regelmäßig Proben entnommen und die Zusammensetzung mittels HPLC analysiert. Figur 4 zeigt die erhaltenen Ergebnisse bezüglich des Fructoseumsatzes, der Selektivitäten und der Kohlenstoffbilanz (Kohlenstoffbilanz = (∑ [nicht umgesetzte Zucker, HMF und Ameisensäure (in Mol)] ^{∗}100 / eingesetzter Zucker (in Mol))). Glukose wurde unter den gewählten Reaktionsbedingungen nicht signifikant umgesetzt, daher beziehen sich die Selektivitäten auf die umgesetzte Fructose.

Die Zusammensetzung der Produktmischung war über den gesamten Versuchszeitraum konstant. Der Fructoseumsatz (8) lag bei etwa 20 %, die HMF-Selektivität (7) bei etwa 83 % und die Kohlenstoffbilanz (6) bei 97 %. Die fehlenden 3 % in der Kohlenstoffbilanz setzten sich aus analytisch nicht erfassten Zwischen- und Nebenprodukten, wie unter anderem löslichen und unlöslichen Huminstoffe, zusammen.

### Beispiel 2: HMF-Synthese mit 20 % Kohlenhydratlösung bei 150 °C mit 0,75 % Säure

Eine 20 Gew.-% Fructoselösung (gemäß A, vorstehend) (80% Fructose, 20% Glukose) in 0,75 Gew.-% Schwefelsäure wurde zunächst auf eine Temperatur von 80 °C vorgeheizt und dann in Schritt c) bei einer Temperatur von 150 °C mit einer Verweilzeit (bezogen auf die Heizzone) von 6 min über eine Versuchsdauer von 40 h kontinuierlich umgesetzt. Im Reaktionsverlauf wurden regelmäßig Proben entnommen und die Zusammensetzung mittels HPLC analysiert. Figur 5 zeigt die erhaltenen Ergebnisse bezüglich des Fructoseumsatzes, der Selektivitäten und der Kohlenstoffbilanz.

Über den gesamten Versuch war die Zusammensetzung der Produktmischung konstant. Der Fructoseumsatz (8) lag bei etwa 19 %, die HMF-Selektivität (7) bei etwa 84 % und die Kohlenstoffbilanz (6) bei 98,6 %. Die fehlenden 1,4 % in der Kohlenstoffbilanz setzen sich aus analytisch nicht erfassten Zwischen- und Nebenprodukten, wie unter anderem die löslichen und unlöslichen Huminstoffe, zusammen.

### Beispiel 3: HMF-Synthese mit 15 % Kohlenhydratlösung bei 153 °C mit 0,5 % Säure

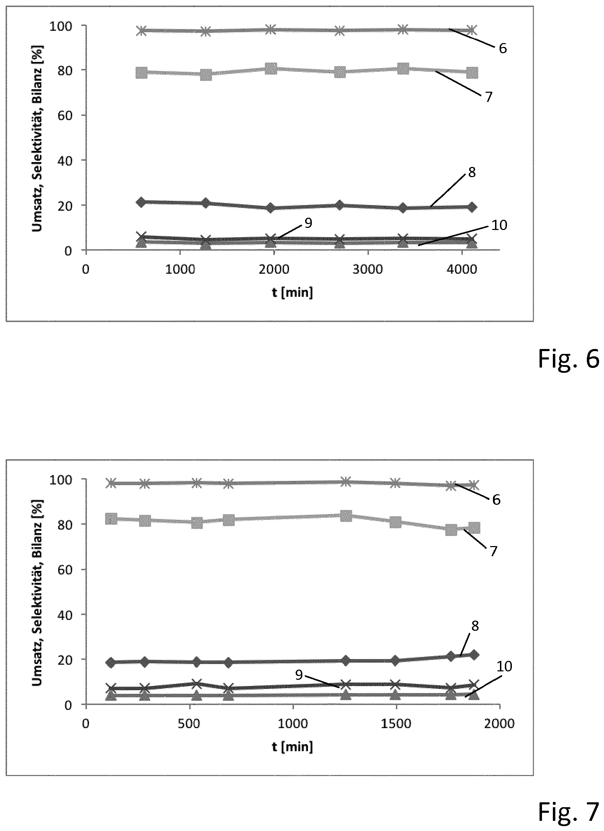

Eine 15 Gew.-% Fructoselösung (gemäß A, vorstehend) (80 % Fructose, 20 % Glukose) in 0,5 Gew.-% Schwefelsäure wurde zunächst auf eine Temperatur von 75 °C vorgeheizt und dann in Schritt c) bei einer Temperatur von 153 °C mit einer Verweilzeit (bezogen auf die Heizzone) von 7 min über eine Versuchsdauer von 68 h kontinuierlich umgesetzt. Im Reaktionsverlauf wurden regelmäßig Proben entnommen und die Zusammensetzung mittels HPLC analysiert. Figur 6 zeigt die erhaltenen Ergebnisse bezüglich des Fructoseumsatzes, der Selektivitäten und der Kohlenstoffbilanz.

Über den gesamten Versuch war die Zusammensetzung der Produktmischung konstant. Der Fructoseumsatz (8) lag bei etwa 20 %, die HMF-Selektivität (7) bei etwa 80 % und die Kohlenstoffbilanz (6) bei 98 %. Die fehlenden 2 % in der Kohlenstoffbilanz setzen sich aus analytisch nicht erfassten Zwischen- und Nebenprodukten, wie unter anderem die löslichen und unlöslichen Huminstoffe, zusammen.

### Beispiel 4: HMF-Synthese mit 25 % Kohlenhydratlösung bei 150 °C mit 0,75 % Säure

Eine 25 Gew.-% Fructoselösung (gemäß A, vorstehend) (80 % Fructose, 20 % Glukose) in 0,75 Gew.-% Schwefelsäure wurde zunächst auf 80 °C vorgeheizt und dann in Schritt c) bei einer Temperatur von 150 °C mit einer Verweilzeit (bezogen auf die Heizzone) von 6 min über eine Versuchsdauer von 31 h kontinuierlich umgesetzt. Im Reaktionsverlauf wurden regelmäßig Proben entnommen und die Zusammensetzung mittels HPLC analysiert. Figur 7 zeigt die erhaltenen Ergebnisse bezüglich des Fructoseumsatzes, der Selektivitäten und der Kohlenstoffbilanz.

Über den gesamten Versuch war die Zusammensetzung der Produktmischung konstant. Der Fructoseumsatz (8) lag bei etwa 19,5 %, die HMF-Selektivität (7) bei etwa 81 % und die Kohlenstoffbilanz (6) bei 98 %. Die fehlenden 2 % in der Kohlenstoffbilanz setzen sich aus analytisch nicht erfassten Zwischen- und Nebenprodukten, wie unter anderem die löslichen und unlöslichen Huminstoffe, zusammen.

### Beispiel 5: HMF-Synthese mit 15 (20) % Kohlenhydratlösung bei 140 °C mit 1,0 % Säure

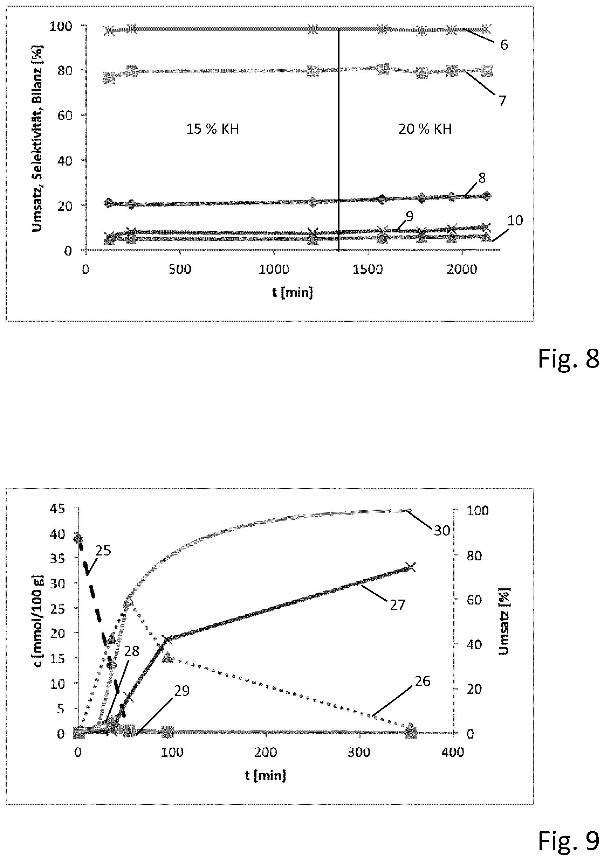

Eine 15 Gew.-% Fructoselösung (gemäß A, vorstehend) (80 % Fructose, 20 % Glukose) in 1,0 Gew.-% Schwefelsäure wurde zunächst auf 75 °C vorgeheizt und anschließend in Schritt c) bei einer Temperatur von 140 °C mit einer Verweilzeit (bezogen auf die Heizzone) von 9 min über eine Versuchsdauer von zunächst 23 h kontinuierlich umgesetzt. Nach 23 h wurde die Kohlenhydratkonzentration im Feed auf 20 Gew.-% erhöht und die Reaktion weitere 12 h unter ansonsten unveränderten Bedingungen fortgesetzt. Im Reaktionsverlauf wurden regelmäßig Proben entnommen und die Zusammensetzung mittels HPLC analysiert. Figur 8 zeigt die erhaltenen Ergebnisse bezüglich des Fructoseumsatzes, der Selektivitäten und der Kohlenstoffbilanz.

Über den gesamten Versuch war die Zusammensetzung der Produktmischung konstant. Der Fructoseumsatz (8) lag bei etwa 19,5 %, die HMF-Selektivität (7) bei etwa 81% und die Kohlenstoffbilanz (6) bei 98 %. Die fehlenden 2 % in der Kohlenstoffbilanz setzen sich aus analytisch nicht erfassten Zwischen- und Nebenprodukten, wie unter anderem die löslichen und unlöslichen Huminstoffe, zusammen.

### Beispiel 6: Kontinuierliche Auftrennung der HMF-haltigen Produktmischung in einem zweistufigen Chromatographieverfahren

### I) Abtrennung einer HMF-Fraktion in einer ersten Stufe und Auftrennung der restlichen Fraktionen in einer zweiten Stufe

Eine in Schritt d) erhaltene Produktmischung wurde auf einen niedrigen Trockensubstanzanteil von 17 Gew.-% eingestellt. Die Produktmischung umfasste 7,8 Gew.-% HMF, 1,8 Gew.-% Lävulinsäure, < 0,1 Gew.-% Ameisensäure, 63,9 Gew.-% Fructose, 22,5 Gew.-% Glukose und 4 Gew.-% Säure, wird in Schritt e) kontinuierlich in einem zweistufigen ChromatographieVerfahren bei 60 °C in vier Fraktionen getrennt.

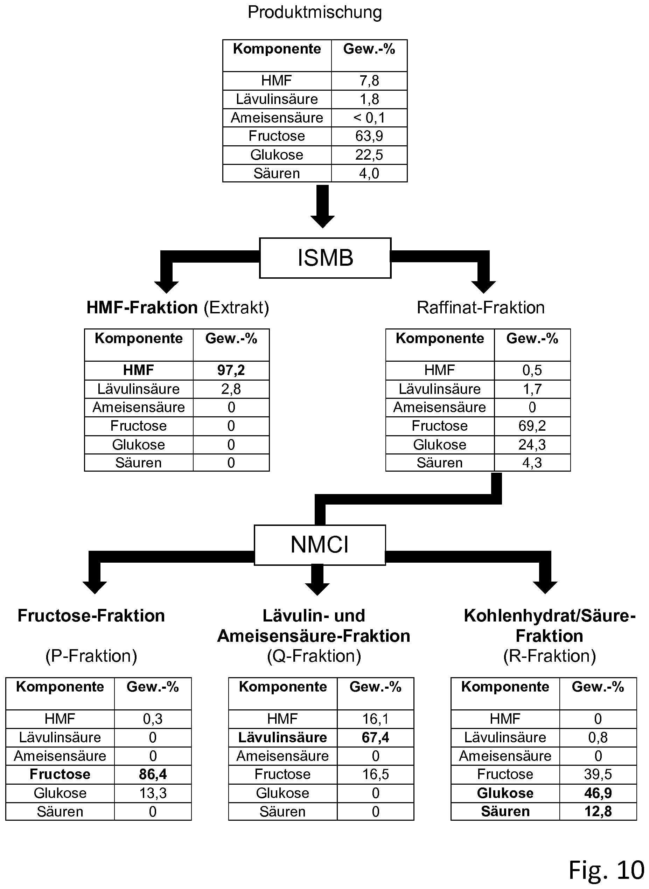

In der ersten Stufe wird die Produktmischung über ein ISMB System an 50 l Diaion UBK 530 Chromatographieharz (Fa. Mitsubishi Chemical) in H⁺-Form mit Wasser als Elutionsmittel und einem Wasser/Produktmischung-Verhältnis von 1,84 in eine HMF-Fraktion (Extrakt) sowie eine Raffinat-Fraktion getrennt (Fig. 10).

Für die HMF-Fraktion ergaben sich folgende Rückgewinnungsraten (bezogen auf den jeweiligen Gehalt der aus Schritt d) zugeführten Produktmischung): HMF: 93,9%, Lävulinsäure: 12,0%, Ameisensäure: 0,0%, Fructose: 0,0%, Glukose: 0,0%, Säure: 0,0%.

Die Raffinat-Fraktion wird anschließend nach Konzentration in der zweiten Stufe über ein NMCI System an 141 l Diaion UBK 530 Chromatographieharz in H⁺-Form mit Wasser als Elutionsmittel und einem Wasser/Produktmischung-Verhältnis von 4,51 in eine Fructose-Fraktion (P-Fraktion), eine Lävulin- und Ameisensäure-Fraktion (Q-Fraktion) sowie eine Kohlenhydrat/Säure-Fraktion bzw. Glukose-Fraktion (R-Fraktion) getrennt (Fig. 10).
Für die Fructose-Fraktion ergaben sich folgende Rückgewinnungsraten (bezogen auf den jeweiligen Gehalt der aus Schritt d) zugeführten Produktmischung): HMF: 2,0%, Lävulinsäure: 0,0%, Ameisensäure: 0,0%, Fructose: 80,3%, Glukose: 35,2%, Säure: 0,0%.

Für die Lävulin- und Ameisensäure-Fraktion ergaben sich folgende Rückgewinnungsraten (bezogen auf den jeweiligen Gehalt der aus Schritt d) zugeführten Produktmischung): HMF: 4,0%, Lävulinsäure: 73,7%, Ameisensäure: 0,0%, Fructose: 0,5%, Glukose: 0,0%, Säure: 0,0%.

Für die Kohlenhydrat/Säure-Fraktion ergaben sich folgende Rückgewinnungsraten (bezogen auf den jeweiligen Gehalt der aus Schritt d) zugeführten Produktmischung): HMF: 0,0%, Lävulinsäure: 14,4%, Ameisensäure: 0,0%, Fructose: 19,2%, Glukose: 64,0%, Säure: 100,0%.

### II) Abtrennung einer Kohlenhydrat/Säure-Fraktion in einer ersten Stufe und Auftrennung der restlichen Fraktionen in einer zweiten Stufe

Eine in Schritt d) erhaltene Produktmischung wurde auf einen niedrigen Trockensubstanzanteil von 17 Gew.-% eingestellt. Die Produktmischung umfasste 7,8 Gew.-% HMF, 1,8 Gew.-% Lävulinsäure, < 0,1 Gew.-% Ameisensäure, 63,9 Gew.-% Fructose, 22,5 Gew.-% Glukose und 4 Gew.-% Salze, wird in Schritt e) kontinuierlich in einem zweistufigen ChromatographieVerfahren bei 60 °C in vier Fraktionen getrennt.

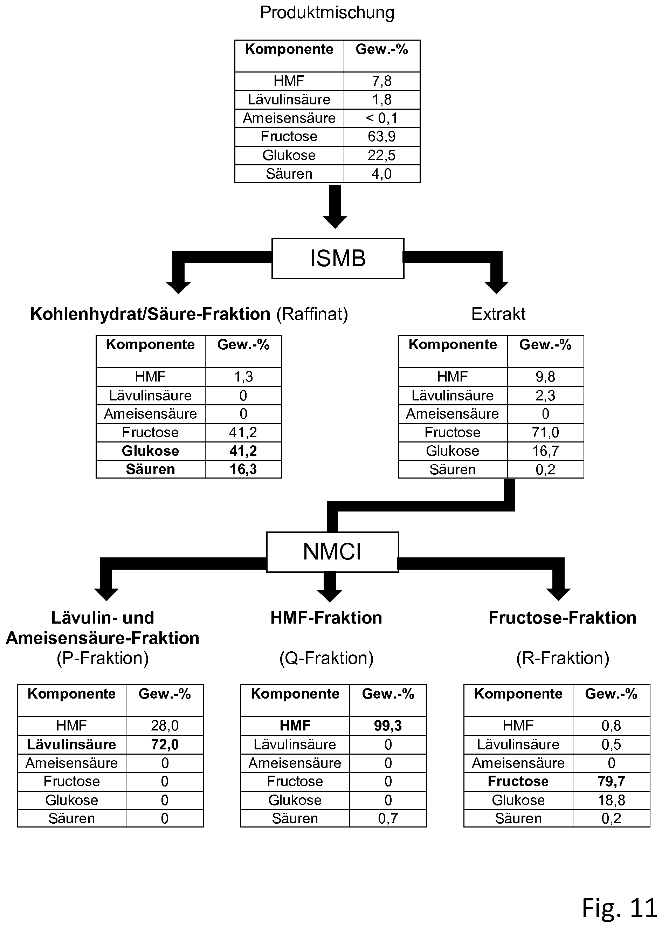

In der ersten Stufe wird das Feed über ein ISMB System an 135 l Diaion UBK 530 Chromatographieharz (Fa. Mitsubishi Chemical) in H⁺-Form mit Wasser als Elutionsmittel und einem Wasser/Produktmischung-Verhältnis von 3,111 in eine Kohlenhydrat/Säure-Fraktion (Raffinat) sowie eine Extrakt-Fraktion getrennt (Fig. 11).

Für die Kohlenhydrat/Säure-Fraktion ergaben sich folgende Rückgewinnungsraten (bezogen auf den jeweiligen Gehalt der aus Schritt d) zugeführten Produktmischung): HMF: 4,0%, Lävulinsäure: 0,0%, Ameisensäure: 0,0%, Fructose: 15,1%, Glukose: 43,1%, Säure: 95,9%.

Die Extrakt-Fraktion wird anschließend nach Konzentration in der zweiten Stufe über ein NMCI System an 190 l Diaion UBK 530 Chromatographieharz in H⁺-Form mit Wasser als Elutionsmittel und einem Wasser/Produktmischung-Verhältnis von 7,49 in die Lävulin- und Ameisensäure-Fraktion (P-Fraktion), die HMF-Fraktion (Q-Fraktion) sowie die Fructose-Fraktion (R-Fraktion) getrennt (Fig. 11).

Für die Lävulin- und Ameisensäure-Fraktion ergaben sich folgende Rückgewinnungsraten (bezogen auf den jeweiligen Gehalt der aus Schritt d) zugeführten Produktmischung): HMF: 6,9%, Lävulinsäure: 79,0%, Ameisensäure: 0,0%, Fructose: 0,0%, Glukose: 0,0%, Säure: 0,0%.

Für die HMF-Fraktion ergaben sich folgende Rückgewinnungsraten (bezogen auf den jeweiligen Gehalt der aus Schritt d) zugeführten Produktmischung): HMF: 82,2%, Lävulinsäure: 0,0%, Ameisensäure: 0,0%, Fructose: 0,0%, Glukose: 0,0%, Säure: 1,3%.

Für die Fructose-Fraktion ergaben sich folgende Rückgewinnungsraten (bezogen auf den jeweiligen Gehalt der aus Schritt d) zugeführten Produktmischung): HMF: 6,9%, Lävulinsäure: 21,0%, Ameisensäure: 0,0%, Fructose: 84,9%, Glukose: 57,0%, Säure: 2,8%.

### III) Abtrennung einer HMF-Fraktion in einer ersten Stufe und Auftrennung der restlichen Fraktionen in einer zweiten Stufe

Eine in Schritt d) erhaltene Produktmischung wurde auf einen hohen Trockensubstanzanteil von 55 Gew.-% eingestellt. Die Produktmischung umfasste 7,6 Gew.-% HMF, 1,7 Gew.-% Lävulinsäure, < 0,1 Gew.-% Ameisensäure, 62,8 Gew.-% Fructose, 23,3 Gew.-% Glukose und 4,6 Gew.-% Salze, wird in Schritt e) kontinuierlich in einem zweistufigen ChromatographieVerfahren bei 60 °C in vier Fraktionen getrennt.

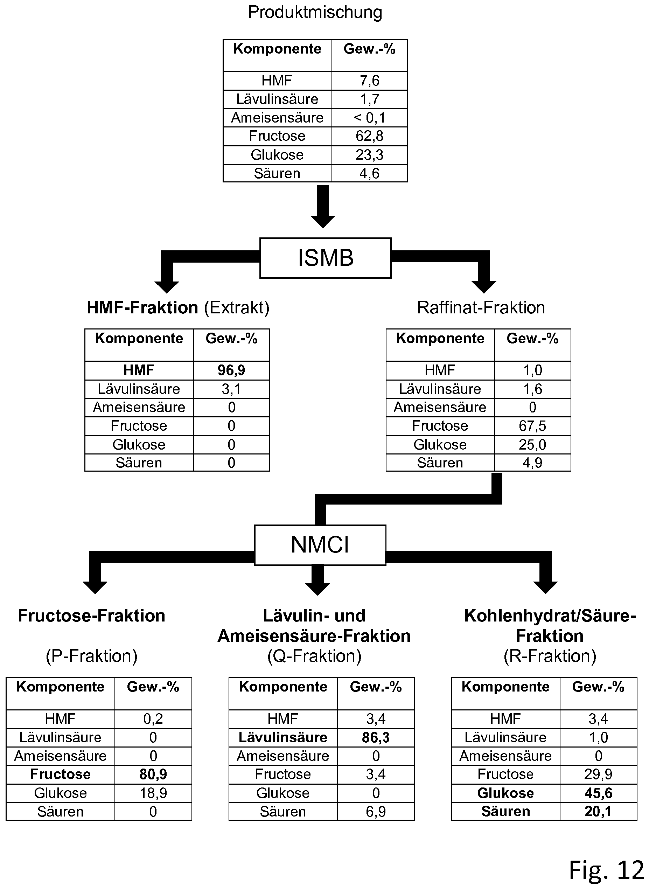

In der ersten Stufe wird die Produktmischung über ein ISMB System an 14 l Diaion UBK 530 Chromatographieharz (Fa. Mitsubishi Chemical) in H⁺-Form mit Wasser als Elutionsmittel und einem Wasser/Produktmischung-Verhältnis von 1,84 in eine HMF-Fraktion (Extrakt) sowie eine Raffinat-Fraktion getrennt (Fig. 12).

Für die HMF-Fraktion ergaben sich folgende Rückgewinnungsraten (bezogen auf den jeweiligen Gehalt der aus Schritt d) zugeführten Produktmischung): HMF: 87,5%, Lävulinsäure: 12,7%, Ameisensäure: 0,0%, Fructose: 0,0%, Glukose: 0,0%, Säure: 0,0%.

Die Raffinat-Fraktion wird anschließend nach Konzentration in der zweiten Stufe über ein NMCI System an 104 l Diaion UBK 530 Chromatographieharz in H⁺-Form mit Wasser als Elutionsmittel und einem Wasser/Produktmischung-Verhältnis von 5,42 in eine Fructose-Fraktion (P-Fraktion), eine Lävulin- und Ameisensäure-Fraktion (Q-Fraktion) sowie eine Kohlenhydrat/Säure-Fraktion (R-Fraktion) getrennt (Fig. 12).

Für die Fructose-Fraktion ergaben sich folgende Rückgewinnungsraten (bezogen auf den jeweiligen Gehalt der aus Schritt d) zugeführten Produktmischung): HMF: 1,8%, Lävulinsäure: 0,0%, Ameisensäure: 0,0%, Fructose: 89,4%, Glukose: 56,4%, Säure: 0,0%.

Für die Lävulin- und Ameisensäure-Fraktion ergaben sich folgende Rückgewinnungsraten (bezogen auf den jeweiligen Gehalt der aus Schritt d) zugeführten Produktmischung): HMF: 0,7%, Lävulinsäure: 74,8%, Ameisensäure: 0,0%, Fructose: 0,1%, Glukose: 0,0%, Säure: 2,2%.

Für die Kohlenhydrat/Säure-Fraktion ergaben sich folgende Rückgewinnungsraten (bezogen auf den jeweiligen Gehalt der aus Schritt d) zugeführten Produktmischung): HMF: 9,9%, Lävulinsäure: 13,3%, Ameisensäure: 0,0%, Fructose: 10,6%, Glukose: 43,8%, Säure: 97,9%.

### IV) Abtrennung einer Kohlenhydrat/Säure-Fraktion in einer ersten Stufe und Auftrennung der restlichen Fraktionen in einer zweiten Stufe

Eine in Schritt d) erhaltene Produktmischung wurde auf einen hohen Trockensubstanzanteil von 55 Gew.-% eingestellt. Die Produktmischung umfasste 7,6 Gew.-% HMF, 1,7 Gew.-% Lävulinsäure, < 0,1 Gew.-% Ameisensäure, 62,8 Gew.-% Fructose, 23,3 Gew.-% Glukose und 4,6 Gew.-% Salze, wird in Schritt e) kontinuierlich in einem zweistufigen ChromatographieVerfahren bei 60 °C in vier Fraktionen getrennt.

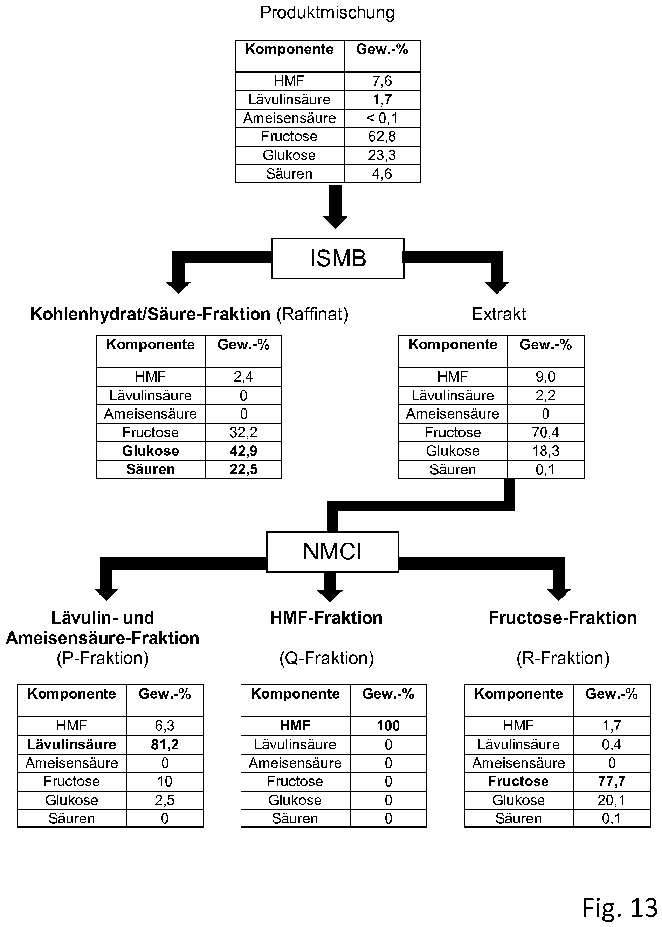

In der ersten Stufe wird das Feed über ein ISMB System an 39 l Diaion UBK 530 Chromatographicharz (Fa. Mitsubishi Chcmical) in H⁺-Form mit Wasser als Elutionsmittel und einem Wasser/Produktmischung-Verhältnis von 3,67 in eine Kohlenhydrat/Säure-Fraktion (Raffinat) sowie eine Extrakt-Fraktion getrennt (Fig. 13).

Für die Kohlenhydrat/Säure-Fraktion ergaben sich folgende Rückgewinnungsraten (bezogen auf den jeweiligen Gehalt der aus Schritt d) zugeführten Produktmischung): HMF: 6,4%, Lävulinsäure: 0,0%, Ameisensäure: 0,0%, Fructose: 10,4%, Glukose: 37,3%, Säure: 99,1%.

Die Extrakt-Fraktion wird anschließend nach Konzentration in der zweiten Stufe über ein NMCI System an 105 l Diaion UBK 530 Chromatographieharz in H⁺-Form mit Wasser als Elutionsmittel und einem Wasser/Produktmischung-Verhältnis von 7,49 in die Lävulin- und Ameisensäure-Fraktion (P-Fraktion), die HMF-Fraktion (Q-Fraktion) sowie die Fructose-Fraktion (R-Fraktion) getrennt (Fig. 13).

Für die Lävulin- und Ameisensäure-Fraktion ergaben sich folgende Rückgewinnungsraten (bezogen auf den jeweiligen Gehalt der aus Schritt d) zugeführten Produktmischung): HMF: 1,4%, Lävulinsäure: 82,2%, Ameisensäure: 0,0%, Fructose: 0,3%, Glukose: 0,2%, Säure: 0,0%.

Für die HMF-Fraktion ergaben sich folgende Rückgewinnungsraten (bezogen auf den jeweiligen Gehalt der aus Schritt d) zugeführten Produktmischung): HMF: 76,4%, Lävulinsäure: 0,0%, Ameisensäure: 0,0%, Fructose: 0,0%, Glukose: 0,0%, Säure: 0,0%.

Für die Fructose-Fraktion ergaben sich folgende Rückgewinnungsraten (bezogen auf den jeweiligen Gehalt der aus Schritt d) zugeführten Produktmischung): HMF: 16,1%, Lävulinsäure: 18,2%, Ameisensäure: 0,0%, Fructose: 89,7%, Glukose: 62,8%, Säure: 1,0%.

### Beispiel 7: Oxidation einer HMF-Lösung aus dem erfindungsgemäßen Prozess

Eine aus einer 10 Gew.-%-igen Fructoselösung (96 % Fructose, 2,3 % Glukose, 1,7 % Restsaccharide) erhaltene Produktmischung wurde chromatographisch aufgetrennt. Die dabei erhaltene HMF-Fraktion hat eine Reinheit von 98,1 g/100 g Trockensubstanz und wurde auf eine Konzentration von 0,4 mol/l aufkonzentriert und in einem Oxidationsversuch unter folgenden Reaktionsbedingungen zu 2,5-Furandicarbonsäure (FDCA) umgesetzt.

Ansatzgröße = 500 g
c_{HMF} = 0,4 mol/l
c_{Katalysator} = 5,94 g/l (bezogen auf die Trockensubstanz des Katalysators)
Katalysator = 5 % Pt/1 % Bi/C (Degussa/Evonik)
T = 60 °C
pH = 9,0
Titrationsmittel = 16 Gew.-% NaOH
Druck = atmosphärisch
O₂ = 500 ml/min

Es wurden regelmäßig Proben entnommen und diese mittels HPLC (BIORAD, Aminex 87-H, 5 mmol/l H₂SO₄, 50°C) auf ihre Zusammensetzung untersucht. Die Versuchsergebnisse sind in Figur 9 dargestellt.

Das eingesetzte HMF (25) konnte vollständig umgesetzt werden. Aus der oxidierten Lösung wurde anschließend die freie 2,5-Furandicarbonsäure (27) durch Absenken des pH-Werts mit Salzsäure ausgefällt, getrocknet und analysiert. Die Reinheit der dabei erhaltenen FDCA betrug 98,2 %.

## Patentansprüche

1. Verfahren zur Herstellung von 5-Hydroxymethylfurfural (HMF) in einem kontinuierlichen Prozess, umfassend die Schritte:
a) Bereitstellen einer wässrigen Fructose-haltigen Ausgangslösung und mindestens eines homogenen sauren Katalysators,
b) Vermischen der wässrigen Fructose-haltigen Ausgangslösung und des mindestens einen homogenen sauren Katalysators zum Erhalt einer Reaktionslösung mit einem Kohlenhydratgehalt von 5 Gew.-% bis 50 Gew.-% (Trockensubstanz Kohlenhydrat in Bezug auf Gesamtgewicht Reaktionslösung) und einem Fructosegehalt von 40 Gew.-% bis 100 Gew.-% (Trockensubstanz Fructose in Bezug auf Trockensubstanz der Kohlenhydrate),
c) Einspeisen der in Schritt b) erhaltenen Reaktionslösung in ein kontinuierliches Reaktorsystem und Umsetzen der in der Reaktionslösung vorhandenen Fructose zu HMF bei einer Temperatur von 80 °C bis 165 °C zum Erhalt einer HMF-haltigen Produktmischung unter Einstellung eines Fructose-Umsatzes von 1 mol-% bis 40 mol-%,
d) Einstellen der Produktmischung auf eine Temperatur von 20 °C bis 80 °C,
e) Aufreinigen der in Schritt d) erhaltenen Produktmischung unter Verwendung einer Chromatographie zur Auftrennung von mindestens vier Fraktionen, umfassend eine HMF-Fraktion, eine Kohlenhydrat/Säure-Fraktion, eine Fructose-Fraktion und eine Lävulin- und Ameisensäure-Fraktion.

2. Verfahren nach Anspruch 1, wobei in Schritt a) eine wässrige Fructose-haltige Ausgangslösung, eine wässrige rückgeführte Fructose-haltige Fraktion und mindestens ein homogener saurer Katalysator bereitgestellt werden, in Schritt b) die wässrige Fructose-haltigen Ausgangslösung, die wässrige rückgeführte Fructose-haltige Fraktion und der mindestens eine homogene saure Katalysator zum Erhalt einer Reaktionslösung mit einem Kohlenhydratgehalt von 5 Gew.-% bis 50 Gew.-% (Trockensubstanz Kohlenhydrat in Bezug auf Gesamtgewicht Reaktionslösung) und einem Fructosegehalt von 40 Gew.-% bis 100 Gew.-% (Trockensubstanz Fructose in Bezug auf Trockensubstanz der Kohlenhydrate) vermischt werden, und wobei die in Schritt e) aufgetrennte oder Schritt f) erhaltene Fructose-Fraktion kontinuierlich zumindest teilweise in den Schritt a) rückgeführt wird.

3. Verfahren nach Anspruch 1 oder 2, wobei die in Schritt b) erhaltene Reaktionslösung auf eine Temperatur von 80 °C bis 165 °C vorgeheizt wird.

4. Verfahren nach Anspruch 1 oder 2, wobei vor Schritt b) mindestens eine der in Schritt a) bereitgestellten Komponenten auf eine Temperatur von 80 °C bis 165 °C vorgeheizt wird.

5. Verfahren nach einem der Ansprüche 1 bis 4, wobei die Konzentration des mindestens einen homogenen sauren Katalysators 0,5 Gew.-% bis 5 Gew.-% (Gew.-% in Bezug auf Gesamtgewicht Reaktionslösung) beträgt.

6. Verfahren nach einem der vorherigen Ansprüche, wobei der mindestens eine homogene saure Katalysator ausgewählt ist aus Schwefelsäure, Salzsäure, Phosphorsäure, aliphatischen oder aromatischen Carbonsäuren und aliphatischen oder aromatischen Sulfonsäuren.

7. Verfahren nach einem der vorherigen Ansprüche, wobei in dem Verfahren keine organischen Lösungsmittel verwendet werden.

8. Verfahren nach einem der vorherigen Ansprüche, wobei die Umsetzung der Fructose zu HMF in Schritt c) in einem Zeitraum von 0,1 bis 20 min erfolgt.

9. Verfahren nach einem der vorherigen Ansprüche, wobei die HMF-Selektivität in Schritt c) 60 mol-% bis 100 mol-% beträgt.

10. Verfahren nach einem der vorherigen Ansprüche, wobei die Chromatographie eine Chromatographie an Ionenaustauscherharzen ist.

11. Verfahren nach einem der vorherigen Ansprüche, wobei es sich bei der Chromatographie, insbesondere Chromatographie an Ionenaustauscherharzen, in Schritt e) um ein Simulated Moving Bed-Verfahren (SMB), ein Sequential Simulated Moving Bed-Verfahren (SSMB), ein Improved Simulated Moving Bed-Verfahren (ISMB) oder ein New MCI-Verfahren (NMCI) handelt.

12. Verfahren nach einem der vorherigen Ansprüche, wobei die Chromatographie in Schritt e) ein mehrstufiges Verfahren ist.

13. Verfahren nach einem der vorherigen Ansprüche, wobei die Chromatographie in Schritt e) eine Chromatographie an Kationenaustauscherharzen ist.

14. Verfahren nach Anspruch 13, wobei die Chromatographie an Kationenaustauscherharzen in Schritt e) unter Verwendung eines Kationenaustauscherharzes in H⁺-Form durchgeführt wird.

15. Verfahren nach einem der vorherigen Ansprüche, wobei der Chromatographie eine Filtration der Produktmischung, eine Entfärbung und/oder eine Reinigung der Produktmischung über Aktivkohle vorgeschaltet sind.

16. Verfahren nach einem der vorherigen Ansprüche, wobei Schritt e) bei einer Temperatur von 40 °C bis 80 °C durchgeführt wird.

17. Verfahren nach einem der vorherigen Ansprüche, wobei die in Schritt d) erhaltene Produktmischung vor Schritt e) auf einen Trockensubstanzgehalt von 20 Gew.-% bis 50 Gew.-% konzentriert wird.

18. Verfahren nach einem der vorherigen Ansprüche, wobei die in Schritt e) aufgetrennte oder in Schritt f) erhaltene Kohlenhydrat/Säure-Fraktion für die Ethanolherstellung eingesetzt wird.

19. Verfahren nach einem der vorherigen Ansprüche, wobei die in Schritt e) aufgetrennte oder in Schritt f) erhaltene Lävulin- und Ameisensäure-Fraktion zur Isolierung von Lävulin- und Ameisensäure eingesetzt wird.

20. Verfahren nach einem der vorherigen Ansprüche, wobei das in Schritt e) aufgetrennte oder in Schritt f) erhaltene HMF direkt und ohne die Notwendigkeit aufwendiger weiterer Aufreinigung in einem weiteren Schritt zu 2,5-Furandicarbonsäure (FDCA) oxidiert wird.

## Claims

1. Method for the production of 5-hydroxymethylfurfural (HMF) in a continuous process comprising the following steps:
a) providing of an aqueous fructose-containing starting solution and at least one homogeneous acid catalyst,
b) mixing of the aqueous fructose-containing starting solution and the at least one homogeneous acid catalyst to obtain a reaction solution having a carbohydrate content ranging from 5 wt-% to 50 wt-% (dry matter carbohydrate relative to the total weight of the reaction solution) and a fructose content ranging from 40 wt-% to 100 wt- % (dry matter fructose relative to the dry matter of the carbohydrates),
c) feeding in of the reaction solution obtained in step b) into a continuous reactor system and conversion of the fructose present in the reaction solution to HMF at a temperature ranging from 80 °C to 165 °C to obtain an HMF-containing product mixture while adjusting for a fructose conversion ranging from 1 mol-% to 40 mol-%,
d) adjusting of the product mixture to a temperature ranging from 20 °C to 80 °C,
e) purificating of the product mixture obtained in step d) by using chromatography to separate at least four fractions comprising an HMF fraction, a carbohydrate/acid fraction, a fructose fraction and a levulinic and formic acid fraction.

2. Method according to claim 1, wherein in step a) an aqueous fructose-containing starting solution, an aqueous recycled fructose-containing fraction and at least one homogeneous acid catalyst are provided, wherein in step b) the aqueous fructose-containing starting solution, the aqueous recycled fructose-containing fraction and the at least one homogeneous acid catalyst are mixed to obtain a reaction solution having a carbohydrate content ranging from 5 wt-% to 50 wt-% (dry matter carbohydrate relative to the total weight of the reaction solution) and a fructose content ranging from 40 wt-% to 100 wt-% (dry matter, fructose in relation to the dry matter of the carbohydrates), and wherein the fructose fraction obtained in step e) or step f) is continuously recycled at least partially to step a).

3. Method according to claim 1 or 2, wherein the reaction solution obtained in step b) is preheated to a temperature ranging from 80 °C to 165 °C.

4. Method according to claim 1 or 2, wherein before step b) at least one of the components provided in step a) is preheated to a temperature ranging from 80 °C to 165 °C.

5. Method according to any one of claims 1 to 4, wherein the concentration of the at least one homogeneous acid catalyst is 0.5 wt-% to 5 wt-% (wt-% relative to the total weight of reaction solution).

6. Method according to any one of the preceding claims, wherein the at least one homogeneous acid catalyst is selected from sulfuric acid, hydrochloric acid, phosphoric acid, aliphatic or aromatic carboxylic acids and aliphatic or aromatic sulfonic acids.

7. Method according to any preceding claims, wherein no organic solvents are used in the method.

8. Method according to any one of the preceding claims, wherein the conversion of the fructose to HMF in step c) takes place during a period ranging from 0.1 to 20 min.

9. Method according to any one of the preceding claims, wherein the HMF selectivity in step c) ranges from 60 mol-% to 100 mol-%.

10. Method according to any one of the preceding claims, wherein the chromatography is a chromatography on ion exchange resins.

11. Method according to any one of the preceding claims, wherein the chromatography, in particular the chromatography on ion exchange resins, in step e) is a Simulated Moving Bed method (SMB), a Sequential Simulated Moving Bed method (SSMB), an Improved Simulated Moving Bed Method (ISMB) or a New MCI Method (NMCI).

12. Method according to any one of the preceding claims, wherein the chromatography in step e) is a multi-step process.

13. Method according to any one of the preceding claims, wherein the chromatography in step e) is a chromatography on cation exchange resins.

14. Method according to claim 13, wherein the chromatography on cation exchange resins in step e) is carried out using a cation exchange resin in H⁺ form.

15. Method according to any one of the preceding claims, wherein the chromatography is preceded by a filtration of the product mixture, a decolorization and/or a purification of the product mixture via activated carbon.

16. Method according to any one of the preceding claims, wherein step e) is carried out at a temperature ranging from 40 °C to 80 °C.

17. Method according to any one of the preceding claims, wherein the product mixture obtained in step d) is concentrated to a dry matter content ranging from 20 wt- % to 50 wt- % before step e).

18. Method according to any one of the preceding claims, wherein the carbohydrate/acid fraction separated in step e) or obtained in step f) is used for the production of ethanol.

19. Method according to any one of the preceding claims, wherein the levulinic and formic acid fraction separated in step e) or obtained in step f) is used for the isolation of levulinic and formic acid.

20. Method according to any one of the preceding claims, wherein the HMF separated in step e) or obtained in step f) is oxidized to 2.5-furandicarboxylic acid (FDCA) directly and without the need for costly further purification in another step.

## Revendications

1. Procédé de préparation de 5-hydroxy méthyl furfural (HMF) dans un procédé en continu, comprenant les étapes :
a) le fait de préparer d'une solution de départ aqueuse contenant du fructose et au moins un catalyseur acide homogène,
b) de mélange de la solution de départ aqueuse contenant du fructose et de l'au moins un catalyseur acide homogène pour l'obtention d'une solution réactionnelle avec une teneur en hydrates de carbone de 5 % en poids à 50 % en poids (hydrates de carbone à l'état de substance sèche par rapport au poids total de solution réactionnelle) et une teneur en fructose de 40 % en poids à 100 % en poids (fructose à l'état de substance sèche par rapport aux hydrates de carbone à l'état de substance sèche),
c) d'alimentation de la solution réactionnelle obtenue dans l'étape b) dans un système de réacteur en continu et conversion du fructose présent dans la solution réactionnelle en HMF à une température de 80 °C à 165 °C pour l'obtention d'un mélange de produits contenant de l'HMF, avec un réglage de la conversion du fructose de 1 % mol à 40 % mol,
d) de réglage du mélange de produits à une température de 20 °C à 80 °C,
e) de purification du mélange de produits obtenu dans l'étape d) moyennant l'emploi d'une chromatographie pour la séparation d'au moins quatre fractions, comprenant une fraction d'HMF, une fraction d'hydrates de carbone / d'acide, une fraction de fructose et une fraction d'acide lévulinique et d'acide formique.

2. Procédé selon la revendication 1, dans lequel, dans l'étape a), une solution de départ aqueuse contenant du fructose, une fraction aqueuse contenant du fructose recyclée et au moins un catalyseur acide homogène sont préparés, dans l'étape b) la solution de départ aqueuse contenant du fructose, la fraction aqueuse contenant du fructose recyclée et l'au moins un catalyseur acide homogène sont mélangés pour l'obtention d'une solution réactionnelle avec une teneur en hydrates de carbone de 5 % en poids à 50 % en poids (hydrates de carbone à l'état de substance sèche par rapport au poids total de solution réactionnelle) et une teneur en fructose de 40 % en poids à 100 % en poids (fructose à l'état de substance sèche par rapport aux hydrates de carbone à l'état de substance sèche), et où la fraction de fructose séparée dans l'étape e) ou obtenue dans l'étape f) est au moins partiellement recyclée en continu dans l'étape a).

3. Procédé selon la revendication 1 ou 2, dans lequel la solution réactionnelle obtenue dans l'étape b) est préchauffée à une température de 80 °C à 165 °C.

4. Procédé selon la revendication 1 ou 2, dans lequel les composants préparés avant l'étape b), au moins un des composants préparés dans l'étape a) sont préchauffés à une température de 80 °C à 165 °C.

5. Procédé selon l'une quelconque des revendications 1 à 4, dans lequel la concentration de l'au moins un catalyseur acide homogène est de 0,5 % en poids à 5 % en poids (% en poids par rapport au poids total de la solution réactionnelle).

6. Procédé selon l'une quelconque des revendications précédentes, dans lequel l'au moins un catalyseur acide homogène est choisi parmi l'acide sulfurique, l'acide chlorhydrique, l'acide phosphorique, des acides carboxyliques aliphatiques ou aromatiques et des acides sulfoniques aliphatiques ou aromatiques.

7. Procédé selon l'une quelconque des revendications précédentes, où aucun solvant organique n'est employé dans le procédé.

8. Procédé selon l'une quelconque des revendications précédentes, dans lequel la conversion du fructose en HMF dans l'étape c) a lieu pendant une durée de 0,1 à 20 min.

9. Procédé selon l'une quelconque des revendications précédentes, dans lequel la sélectivité de l'HMF dans l'étape c) est de 60 % mol à 100 % mol.

10. Procédé selon l'une quelconque des revendications précédentes, dans lequel la chromatographie est une chromatographie sur résines échangeuses d'ions.

11. Procédé selon l'une quelconque des revendications précédentes, dans lequel, la chromatographie, en particuler la chromatographie sur résines échangeuses d'ions, dans l'étape e), il s'agit d'un Simulated Moving Bed method (SMB), d'un Sequential Simulated Moving Bed method (SSMB), d'un Improved Simulated Moving Bed method (ISMB) ou d'un New MCI Method (NMCI).

12. Procédé selon l'une quelconque des revendications précédentes, dans lequel la chromatographie dans l'étape e) est un procédé en plusieurs étapes.

13. Procédé selon l'une quelconque des revendications précédentes, dans lequel la chromatographie dans l'étape e) est une chromatographie sur résines échangeuses de cations.

14. Procédé selon la revendication 13, dans lequel la chromatographie sur résine échangeuse de cations dans l'étape e) est effectuée moyennant l'emploi d'une résine d'échange de cations de forme H⁺.

15. Procédé selon l'une quelconque des revendications précédentes, dans lequel la chromatographie est précédée d'une filtration du mélange des produits, d'une décoloration et/ou d'une purification du mélange de produits sur du charbon actif.

16. Procédé selon l'une quelconque des revendications précédentes, dans lequel l'étape e) est effectuée à une température de 40 °C à 80 °C.

17. Procédé selon l'une quelconque des revendications précédentes, dans lequel le mélange de produits obtenu dans l'étape d) est concentré avant l'étape e) jusqu'à une teneur en matière sèche de 20 % en poids à 50 % en poids.

18. Procédé selon l'une quelconque des revendications précédentes, dans lequel la fraction hydrate de carbone / acide séparée dans l'étape e) ou obtenue dans l'étape f) est employée pour la préparation d'éthanol.

19. Procédé selon l'une quelconque des revendications précédentes, dans lequel la fraction d'acide lévulinique et d'acide formique séparée dans l'étape e) ou obtenue dans l'étape f) est employée pour l'isolement d'acide lévulinique et d'acide formique.

20. Procédé selon l'une quelconque des revendications précédentes, dans lequel l'HMF séparé dans l'étape e) ou obtenu dans l'étape f) est oxydé, directement et sans nécessiter de nouvelles purifications laborieuses, en acide 2,5-furan dicarboxylique (FDCA) dans une étape ultérieure.
